# EUROPEAN PATENT SPECIFICATION

(11) **EP 2 435 574 B1**
(45) Date of publication and mention of the grant of the patent: **15.07.2015**
(21) Application number: 10734580.3
(22) Date of filing: 27.05.2010
(51) Int. Cl.: C12N 15/82

(54) **A DNA CASSETTE, BINARY VECTOR, AND STRAIN OF A. TUMEFACIENS AND A METHOD OF PRODUCING CEREAL PLANT OF INCREASED PRODUCTIVITY AND/OR ROOT MASS**
DNA-KASSETTE, BINÄRER VEKTOR UND A. TUMEFACIENS-STAMM SOWIE VERFAHREN ZUR HERSTELLUNG EINER GETREIDEPFLANZE MIT ERHÖHTER PRODUKTIVITÄT UND/ODER WURZELMASSE
CASSETTE D'ADN, VECTEUR BINAIRE, ET SOUCHE D'A. TUMEFACIENS ET PROCÉDÉ DE PRODUCTION D'UNE PLANTE CÉRÉALIÈRE À PRODUCTIVITÉ ACCRUE ET/OU À MASSE DE RACINES PLUS ÉLEVÉE

(30) Priority: 27.05.2009 PL 38811809
(43) Date of publication of application: 04.04.2012
(73) Proprietor: Plant Breeding and Acclimatization Institute - National Research Institute, Blonie (PL)
(72) Inventor: NADOLSKA-ORCZYK, Anna, 05-870 Blonie (PL); GALUSZKA, Petr, 77- Olomouc (CZ); ZALEWSKI, Wojciech, 15-054 Bialystok (PL); ORCZYK, Waclaw, PL-05-870 Blonie (PL)
(74) Representative: Witek, Rafal
(86) International application number: PCT/PL2010/050019
(87) International publication number: WO 2010/138011

(56) References cited:
- WO-A1-2005/123926
- WO-A2-03/050287
- ZALEWSKI WOJCIECH ET AL: "Silencing of the HvCKX1 gene decreases the cytokinin oxidase/dehydrogenase level in barley and leads to higher plant productivity" JOURNAL OF EXPERIMENTAL BOTANY, vol. 61, no. 6, April 2010 (2010-04), pages 1839-1851, XP002599689 ISSN: 0022-0957

### SEQUENCE LISTING

<110> Instytut Hodowli i Aklimatyzacji Roslin Nadolska-Orczyk, Anna Galuszka, Petr Zalewski, Wojciech Orczyk, Waclaw
<120> DNA cassette, binary vector, strain of A. tumefaciens and a method of producing a cereal plant with increased productivity and/or root mass
<130> PZ/776/RW/PCT
<160> 36
<170> PatentIn version 3.3
<210> 1
   <211> 420
   <212> DNA
   <213> artificial sequence
<220>
   <223> CKX fragment
<400> 1
   cctcatccct ggctcaacgt gctcgtgccc cgctccggca tcgccgactt cgaccgcgcc      60
   gtcttcaggg gcatcctcca gggcaccgac atcgccgggc ccctcgtcgt ctacccactc     120
   aacaaatcca agtgggacga cggcatgtcg gcggtgacgc cggcggagga ggtgttctac     180
   gcggtgtcga tgctcttctc gtcggtggcc aacgacctga ggcggctgga ggcgcagaac     240
   cagaagatac tgcggttctg cgacctcgcc gggatagggt acaaggagta cctggcgcac     300
   tacacggccc acggcgactg ggtccggcac ttcggcggca agtggaagca cttcgtggag     360
   atgaaggaca agtacgaccc caagaagctg ctctccccgg gccaagacat cttcaactaa     420
<210> 2
   <211> 1828
   <212> DNA
   <213> artificial sequence
<220>
   <223> CKX fragment
<400> 2
   agtgaaccac taccctgcta cacgcctttc atctttctcc tgaaaacctc accactaaac      60
   ttatttattc ttgacctaca gaagagccat gaggcaatta ctcctgcaat acctgaagct     120
   gttcctgttg ctaggccttg gcgcagtcac tgctgagcat gtgcttaaac atgatgtgct     180
   tgcatccctg gggacgctcc cccttgacgg gcatttcagc ttccacgact tgtctgcagc     240
   tgcaatggac ttcggcaacc tctctagctt cccgccagtc gctgtgcttc acccaggttc     300
   agtggctgac attgccacaa ccgtgaggca tgtgttcttg atgggtgagc actccgcgct     360
   cacagtggca gctcgtgggc atggacactc gctatatggg cagtcccagg ctgctggagg     420
   gattgtcatc agaatggaat cccttcggag tgtcaaaatg caggtgcatc ctggtgcatc     480
   accctatgtg gatgcctcag gaggcgaact ctggataaat gtcttgaata agacgttgaa     540
   gtatggtttg gcgccgaagt catggacaga ctacctccac cttacggttg ggggcacgtt    600
   gtcaaatgcg ggcgtcagcg ggcagacatt ccggcatggt ccacagatca gcaatgtgaa     660
   cgaattggag attgtgactg gaagaggtga tattgtcact tgctcaccag aacagaactc     720
   tgatctcttc cgtgctgctc ttggtggtct gggtcagttt ggcatcatta ctcgggccag     780
   gatcgcactt gagcctgctc cacaaatggt gaggtggata agagttctct acttagattt     840
   catgagcttc accgaggatc aggagatgct tatttcagca gagaagacct tcgactacat     900
   tgaaggtttc gttatcataa acagaacagg catcctaaac aactggaggt catcgttcaa     960
   tccacaggac ccagagcggg ctagccggtt cgaaacagac agaaaagtgc tcttctgcct    1020
   cgagatgaca aagaacttca accctgaaga agctgacatc atggaacagg aggtccatgc    1080
   actactatct caacttagat acacaccagc ctccttattc cacacggacg tcacttacat    1140
   tgagttcttg gatagggtgc actcctctga gatgaagctg agagctaagg gcttgtggga    1200
   agtcccacac ccatggctta atctcatcat accaagaagc actatccata catttgcaga    1260
   gcaggtcttt gggaaaatcc tcgaagataa caacaatggt cccatattgc tctacccagt    1320
   gaagaagtcc agatgggaca accgaacgtc agtggtcata ccagatgagg aagttttcta    1380
   cctggtggga ttcctatcct cggcgatagg cccccacagc atcgaacata cattgaacct    1440
   gaacaaccag ataatagagt tctctaacaa agcaagtatt ggggtgaagc aatatcttcc    1500
   aaactacacc acagaacccg agtggaaggc ccactatggg gctaggtggg acgcatttca    1560
   acagaggaaa aacacctatg accccctggc aatcctagct ccaggacaga aaatatttca    1620
   aaagaaacca gcatcactac ccttgtcctc gttacagtac ctactgtaaa aaatatatat    1680
   gtggagcaat atgtctatgt tagtatggaa ctatagtcgc tttgcaaaag ataacgaact    1740
   gcagcgtgaa ggacactgta cagagtagtg actattagta gtggtgatgc tcaaaatact    1800
   tttagcactg agatcaatga agatcagc                                       1828
<210> 3
   <211> 789
   <212> DNA
   <213> artificial sequence
<220>
   <223> CKX fragment
<400> 3
   ggccgccggg tgaggaactc gggcgggttc ttcaccgacg ccgacgtcgc caggatcgtg      60
   gccgtcgccg cggcgaggaa cgctaccacc gtgtacgtca tcgagacgac gctcaactac     120
   gacagcgcca cggccgcgtc cgtggaccag gagctcagct cggtgctggc gacgctgagg     180
   cacgaggagg ggcacgcgtt cgtgcgggac gcgtcgtacc tggagttcct ggaccgggtg     240
   cacggcgagg agggggcgct ggacaagatc gggctgtggc gtgtcccgca cccttggctc     300
   atcgtgctcg tgccccgctc ccgcatcgcc gacttcgacc gcggcgtctt caagggcatc     360
   ctccagggca ccgacatcgc cgggcctctc gtcgtctacc cgctcaacaa atccaagtac      420
   gcactacgcg tccatcgtgc atgcatgcat gcgatcctgc atggtatgta tcacgcactt     480
   aactgacact ggctcggtcg tggtttcagg tgggacgacg gcatgtcggc ggtgacgcca     540
   gcggaggagg tgttctacgc ggtgtcgctg cccttctcgt cggtggccaa cgacctgaag     600
   cggctggagg cgcagaacca gaagatactg cggttctgcg acctcgccgg ggtagggtac     660
   aaggagtacc tggcgcacta cacggcccac ggcgactggg tccggcactt cggcggcaag     720
   tggcagcgct tcgtggagat gaaggacaag tacgacccca agaggctgct ctccccaggc     780
   caggacatc                                                             789
<210> 4
   <211> 585
   <212> DNA
   <213> artificial sequence
<220>
   <223> TaCKX4 fragment
<400> 4
   gctgtctcgg ctgagataca tacagtcaac cctcttccac accgacgtca cgtacctcga      60
   gttcctggac agggtgcact cctccgagct caagctcagg gcccagggcc tctgggaggt     120
   tccacaccca tggctcaacc tcctcatccc gagaagcacc atccaccggt tcgcgcggga     180
   ggtcttcggc aagatcctga aagacagcaa caatggcccc atactcctct acccagtaaa     240
   cagatcaaag tgggacaaca ggacgtcagt ggtgataccg gaagaagaaa tcttctacct     300
   ggtggggttc ctgtcgtcgg caccgtccgc ctcaggccac ggcagcgtcg agcatgcggt     360
   gagcctgaac gacaagatcg tagacttctg cgacaaggcg ggcgtcggga tgaagcagta     420
   cctagcgccc tacaccaccc agcagcagtg gaaagcccac ttcggggcga ggtgggagac     480
   atttgagcgg aggaaacaca tgtatgatcc cctagcaatc ctagccccag gacagagaat     540
   atttccaaag gcatcactgc ccatgtcctc ttgacaaaca gcatc                     585
<210> 5
   <211> 538
   <212> DNA
   <213> artificial sequence
<220>
   <223> TaCKX7 fragment
<400> 5
   cgtgcaggac gtgtcgtacg cggggttcct tgaccgggtg cgcgacggcg agctcaagct      60
   ccgcgccgcc ggcctctggg acgtgccgca cccctggctc aacctcttcc tcccgcgctc     120
   ccgcgtcctc gacttcgcgg ccggcgtctt ccacggcatc ctccgccgcg acggcggcac     180
   cggccccatg ggccccgtcc tcgtctaccc catgaaccgg gacaggtggg acggcaacac     240
   gtcagcggtg ttcccagagg aggaggaggt gttctacacg gttgggatcc ttcggtcggc     300
   ggtgtccgag ggtgaccttg ggcgtctgga ggagcagaac gaggagatct tacgcttctg     360
   cgaggaggca gggataccgt gcgtgcagta cctgccgtac tacgccgacc aggccgggtg     420
   ggagaagaag cactttggtc cagccaagtg ggccaggttc gtggagcgga agagggagta     480
   tgaccccaag gcgatcctat cccgtggtca gagaattttc acctccccgc tggcttga       538
<210> 6
   <211> 4697
   <212> DNA
   <213> artificial sequence
<220>
   <223> silencing cassette for gene HvCKX1
<400> 6
   aatcccacca aaacctgaac ctagcagttc agttgctcct ctcagagacg aatcgggtat      60
   tcaacaccct cataccaact actacgtcgt gtataacgga cctcatgccg gtatatacga     120
   tgactggggt tgtacaaagg cagcaacaaa cggtgttccc ggagttgcgc ataagaagtt     180
   tgccactatt acagaggcaa gagcagcagc tgacgcgtat acaacaagtc agcaaacaga     240
   taggttgaac ttcatcccca aaggagaagc tcaactcaag cccaagagct ttgcgaaggc     300
   cctaacaagc ccaccaaagc aaaaagccca ctgctcacgc taggaaccaa aaggcccagc     360
   agtgatccag ccccaaaaga gatctccttt gccccggaga ttacaatgga cgatttcctc     420
   tatctttacg atctaggaag gaagttcgaa ggtgaaggtg acgacactat gttcaccact     480
   gataatgaga aggttagcct cttcaatttc agaaagaatg ctgacccaca gatggttaga     540
   gaggcctacg cagcaggtct catcaagacg atctacccga gtaacaatct ccaggagatc     600
   aaataccttc ccaagaaggt taaagatgca gtcaaaagat tcaggactaa ttgcatcaag     660
   aacacagaga aagacatatt tctcaagatc agaagtacta ttccagtatg gacgattcaa     720
   ggcttgcttc ataaaccaag gcaagtaata gagattggag tctctaaaaa ggtagttcct     780
   actgaatcta aggccatgca tggagtctaa gattcaaatc gaggatctaa cagaactcgc     840
   cgtgaagact ggcgaacagt tcatacagag tcttttacga ctcaatgaca agaagaaaat     900
   cttcgtcaac atggtggagc acgacactct ggtctactcc aaaaatgtca aagatacagt     960
   ctcagaagac caaagggcta ttgagacttt tcaacaaagg ataatttcgg gaaacctcct    1020
   cggattccat tgcccagcta tctgtcactt catcgaaagg acagtagaaa aggaaggtgg    1080
   ctcctacaaa tgccatcatt gcgataaagg aaaggctatc attcaagatc tgcctctgcc    1140
   gacagtggtc ccaaagatgg acccccaccc acgaggagca tcgtggaaaa agaagacgtt    1200
   ccaaccacgt cttcaaagca agtggattga tgtgacatct ccactgacgt aagggatgac    1260
   gcacaatccc actatccttc gcaagaccct tcctctatat aaggaagttc atttcatttg    1320
   gagaggacac gctcgagccc ccatttaaat cccccctcga ggatcaagtg caaaggtccg    1380
   ccttgtttct cctctgtctc ttgatctgac taatcttggt ttatgattcg ttgagtaatt    1440
   ttggggaaag ctagcttcgt ccacagtttt tttttcgatg aacagtgccg cagtggcgct    1500
   gatcttgtat gctatcctgc aatcgtggtg aacttatgtc ttttatatcc ttcactacca    1560
   tgaaaagact agctagtaat ctttctcgat gtaacatcgt ccagcactgc tattaccgtg    1620
   tggtccatcc gacagtctgg ctgaacacat catacgatat tgagcaaaga tcgatctatc    1680
   ttccctgttc tttaatgaaa gacgtcattt tcatcagtat gatctaagaa tgttgcaact    1740
   tgcaaggagg cgtttctttc tttgaattta actaactcgt tgagtggccc tgtttctcgg    1800
   acgtaaggcc tttgctgctc cacacatgtc cattcgaatt ttaccgtgtt tagcaagggc    1860
   gaaaagtttg catcttgatg atttagcttg actatgcgat tgctttcctg gacccgtgca    1920
   gctgcggcca tgggagggcg cgccagagat atcagacgga ccgcctcatc cctggctcaa    1980
   cgtgctcgtg ccccgctccg gcatcgccga cttcgaccgc gccgtcttca ggggcatcct    2040
   ccagggcacc gacatcgccg ggcccctcgt cgtctaccca ctcaacaaat ccaagtggga    2100
   cgacggcatg tcggcggtga cgccggcgga ggaggtgttc tacgcggtgt cgatgctctt     2160
   ctcgtcggtg gccaacgacc tgaggcggct ggaggcgcag aaccagaaga tactgcggtt     2220
   ctgcgacctc gccgggatag ggtacaagga gtacctggcg cactacacgg cccacggcga     2280
   ctgggtccgg cacttcggcg gcaagtggaa gcacttcgtg gagatgaagg acaagtacga     2340
   ccccaagaag ctgctctccc cgggccaaga catcttcaac taaagaccta ggaaggtata     2400
   catatatgtt tataattctt tgtttcccct cttattcaga tcgatcacat gcatctttca     2460
   ttgctcgttt ttccttacaa gtagtctcat acatgctaat ttctgtaagg tgttgggctg     2520
   gaaattaatt aattaattaa ttgacttgcc aagatccata tatatgtcct gatattaaat     2580
   cttcgttcgt tatgtttggt taggctgatc aatgttattc tagagtctag agaaacacac     2640
   ccaggggttt tccaactagc tccacaagat ggtgggctag ctgacctaga tttgaagtct     2700
   cactccttat aattatttta tattagatca ttttctaata ttcgtgtctt tttttattct     2760
   agagtctaga tcttgtgttc aactctcgtt aaatcatgtc tctcgccact ggagaaacag     2820
   atcaggaggg tttattttgg gtataggtca aagctaagat tgaaattcac aaatagtaaa     2880
   atcagaatcc aaccaatttt agtagccgag ttggtcaaag gaaaatgtat atagctagat     2940
   ttattgtttt ggcaaaaaaa aatctgaata tgcaaaatac ttgtatatct ttgtattaag     3000
   aagatgaaaa taagtagcag aaaattaaaa aatggattat atttcctggg ctaaaagaat     3060
   tgttgatttg gcacaattaa attcagtgtc aaggttttgt gcaagaattc agtgtgaagg     3120
   aatagattct cttcaaaaca atttaatcat tcatctgatc tgctcaaagc tctgtgcatc     3180
   tccgggtgca acggccagga tatttattgt gcagtaaaaa aatgtcatat cccctagcca     3240
   cccaagaaac tgctccttaa gtccttataa gcacatatgg cattgtaata tatatgtttg     3300
   agttttagcg acaatttttt taaaaacttt tggtcctttt tatgaacgtt ttaagtttca     3360
   ctgtcttttt ttttcgaatt ttaaatgtag cttcaaattc taatccccaa tccaaattgt     3420
   aataaacttc aattctccta attaacatct taattcattt atttgaaaac cagttcaaat     3480
   tcttttaggc tcaccaaacc ttaaacaatt caattcagtg gagctcagag cgatcgcaga     3540
   cccgggaggt tagttgaaga tgtcttggcc cggggagagc agcttcttgg ggtcgtactt     3600
   gtccttcatc tccacgaagt gcttccactt gccgccgaag tgccggaccc agtcgccgtg     3660
   ggccgtgtag tgcgccaggt actccttgta ccctatcccg gcgaggtcgc agaaccgcag     3720
   tatcttctgg ttctgcgcct ccagccgcct caggtcgttg gccaccgacg agaagagcat     3780
   cgacaccgcg tagaacacct cctccgccgg cgtcaccgcc gacatgccgt cgtcccactt     3840
   ggatttgttg agtgggtaga cgacgagggg cccggcgatg tcggtgccct ggaggatgcc     3900
   cctgaagacg gcgcggtcga agtcggcgat gccggagcgg ggcacgagca cgttgagcca     3960
   gggatgagga ctagtgggtc tagagtcctg ctttaatgag atatgcgaga cgcctatgat     4020
   cgcatgatat ttgctttcaa ttctgttgtg cacgttgtaa aaaacctgag catgtgtagc     4080
   tcagatcctt accgccggtt tcggttcatt ctaatgaata tatcacccgt tactatcgta     4140
   tttttatgaa taatattctc cgttcaattt actgattgta ccctactact tatatgtaca     4200
   atattaaaat gaaaacaata tattgtgctg aataggttta tagcgacatc tatgatagag     4260
   cgccacaata acaaacaatt gcgttttatt attacaaatc caattttaaa aaaagcggca     4320
   gaaccggtca aacctaaaag actgattaca taaatcttat tcaaatttca aaagtgcccc     4380
   aggggctagt atctacgaca caccgagcgg cgaactaata acgctcactg aagggaactc     4440
   cggttccccg ccggcgcgca tgggtgagat tccttgaagt tgagtattgg ccgtccgctc     4500
   taccgaaagt tacgggcacc attcaacccg gtccagcacg gcggccgggt aaccgacttg     4560
   ctgccccgag aattatgcag catttttttg gtgtatgtgg gccccaaatg aagtgcaggt     4620
   caaaccttga cagtgacgac aaatcgttgg gcgggtccag ggcgaatttt gcgacaacat     4680
   gtcgaggctc agcagga                                                    4697
<210> 7
   <211> 4467
   <212> DNA
   <213> artificial sequence
<220>
   <223> silencing cassette for gene HvCKX2
<400> 7
   aatcccacca aaacctgaac ctagcagttc agttgctcct ctcagagacg aatcgggtat      60
   tcaacaccct cataccaact actacgtcgt gtataacgga cctcatgccg gtatatacga     120
   tgactggggt tgtacaaagg cagcaacaaa cggtgttccc ggagttgcgc ataagaagtt     180
   tgccactatt acagaggcaa gagcagcagc tgacgcgtat acaacaagtc agcaaacaga     240
   taggttgaac ttcatcccca aaggagaagc tcaactcaag cccaagagct ttgcgaaggc     300
   cctaacaagc ccaccaaagc aaaaagccca ctgctcacgc taggaaccaa aaggcccagc     360
   agtgatccag ccccaaaaga gatctccttt gccccggaga ttacaatgga cgatttcctc     420
   tatctttacg atctaggaag gaagttcgaa ggtgaaggtg acgacactat gttcaccact     480
   gataatgaga aggttagcct cttcaatttc agaaagaatg ctgacccaca gatggttaga     540
   gaggcctacg cagcaggtct catcaagacg atctacccga gtaacaatct ccaggagatc     600
   aaataccttc ccaagaaggt taaagatgca gtcaaaagat tcaggactaa ttgcatcaag     660
   aacacagaga aagacatatt tctcaagatc agaagtacta ttccagtatg gacgattcaa     720
   ggcttgcttc ataaaccaag gcaagtaata gagattggag tctctaaaaa ggtagttcct     780
   actgaatcta aggccatgca tggagtctaa gattcaaatc gaggatctaa cagaactcgc     840
   cgtgaagact ggcgaacagt tcatacagag tcttttacga ctcaatgaca agaagaaaat     900
   cttcgtcaac atggtggagc acgacactct ggtctactcc aaaaatgtca aagatacagt     960
   ctcagaagac caaagggcta ttgagacttt tcaacaaagg ataatttcgg gaaacctcct    1020
   cggattccat tgcccagcta tctgtcactt catcgaaagg acagtagaaa aggaaggtgg    1080
   ctcctacaaa tgccatcatt gcgataaagg aaaggctatc attcaagatc tgcctctgcc    1140
   gacagtggtc ccaaagatgg acccccaccc acgaggagca tcgtggaaaa agaagacgtt    1200
   ccaaccacgt cttcaaagca agtggattga tgtgacatct ccactgacgt aagggatgac    1260
   gcacaatccc actatccttc gcaagaccct tcctctatat aaggaagttc atttcatttg    1320
   gagaggacac gctcgagccc ccatttaaat cccccctcga ggatcaagtg caaaggtccg    1380
   ccttgtttct cctctgtctc ttgatctgac taatcttggt ttatgattcg ttgagtaatt    1440
   ttggggaaag ctagcttcgt ccacagtttt tttttcgatg aacagtgccg cagtggcgct    1500
   gatcttgtat gctatcctgc aatcgtggtg aacttatgtc ttttatatcc ttcactacca    1560
   tgaaaagact agctagtaat ctttctcgat gtaacatcgt ccagcactgc tattaccgtg    1620
   tggtccatcc gacagtctgg ctgaacacat catacgatat tgagcaaaga tcgatctatc    1680
   ttccctgttc tttaatgaaa gacgtcattt tcatcagtat gatctaagaa tgttgcaact    1740
   tgcaaggagg cgtttctttc tttgaattta actaactcgt tgagtggccc tgtttctcgg    1800
   acgtaaggcc tttgctgctc cacacatgtc cattcgaatt ttaccgtgtt tagcaagggc    1860
   gaaaagtttg catcttgatg atttagcttg actatgcgat tgctttcctg gacccgtgca    1920
   gctgcggcca tgggagggcg cgccagagat atcagacgga ccgactagtg aggcgaactc    1980
   tggataaatg tcttgaataa gacgttgaag tatggtttgg cgccgaagtc atggacagac    2040
   tacctccacc ttacggttgg gggcacgttg tcaaatgcgg gcgtcagcgg gcagacattc    2100
   cggcatggtc cacagatcag caatgtgaac gaattggaga ttgtgactgg aagaggtgat    2160
   attgtcactt gctcaccaga acagaactct gatctcttcc gtgctgctct tggtggtctg    2220
   ggtcagtttg agctccctag gaaggtatac atatatgttt ataattcttt gtttcccctc    2280
   ttattcagat cgatcacatg catctttcat tgctcgtttt tccttacaag tagtctcata    2340
   catgctaatt tctgtaaggt gttgggctgg aaattaatta attaattaat tgacttgcca    2400
   agatccatat atatgtcctg atattaaatc ttcgttcgtt atgtttggtt aggctgatca    2460
   atgttattct agagtctaga gaaacacacc caggggtttt ccaactagct ccacaagatg    2520
   gtgggctagc tgacctagat ttgaagtctc actccttata attattttat attagatcat    2580
   tttctaatat tcgtgtcttt ttttattcta gagtctagat cttgtgttca actctcgtta    2640
   aatcatgtct ctcgccactg gagaaacaga tcaggagggt ttattttggg tataggtcaa    2700
   agctaagatt gaaattcaca aatagtaaaa tcagaatcca accaatttta gtagccgagt    2760
   tggtcaaagg aaaatgtata tagctagatt tattgttttg gcaaaaaaaa atctgaatat    2820
   gcaaaatact tgtatatctt tgtattaaga agatgaaaat aagtagcaga aaattaaaaa    2880
   atggattata tttcctgggc taaaagaatt gttgatttgg cacaattaaa ttcagtgtca    2940
   aggttttgtg caagaattca gtgtgaagga atagattctc ttcaaaacaa tttaatcatt    3000
   catctgatct gctcaaagct ctgtgcatct ccgggtgcaa cggccaggat atttattgtg    3060
   cagtaaaaaa atgtcatatc ccctagccac ccaagaaact gctccttaag tccttataag    3120
   cacatatggc attgtaatat atatgtttga gttttagcga caattttttt aaaaactttt    3180
   ggtccttttt atgaacgttt taagtttcac tgtctttttt tttcgaattt taaatgtagc    3240
   ttcaaattct aatccccaat ccaaattgta ataaacttca attctcctaa ttaacatctt    3300
   aattcattta tttgaaaacc agttcaaatt cttttaggct caccaaacct taaacaattc    3360
   aattcagtgg agctcaaact gacccagacc accaagagca gcacggaaga gatcagagtt    3420
   ctgttctggt gagcaagtga caatatcacc tcttccagtc acaatctcca attcgttcac    3480
   attgctgatc tgtggaccat gccggaatgt ctgcccgctg acgcccgcat ttgacaacgt    3540
   gcccccaacc gtaaggtgga ggtagtctgt ccatgacttc ggcgccaaac catacttcaa    3600
   cgtcttattc aagacattta tccagagttc gcctcactag tgggtctaga gtcctgcttt    3660
   aatgagatat gcgagacgcc tatgatcgca tgatatttgc tttcaattct gttgtgcacg    3720
   ttgtaaaaaa cctgagcatg tgtagctcag atccttaccg ccggtttcgg ttcattctaa    3780
   tgaatatatc acccgttact atcgtatttt tatgaataat attctccgtt caatttactg    3840
   attgtaccct actacttata tgtacaatat taaaatgaaa acaatatatt gtgctgaata    3900
   ggtttatagc gacatctatg atagagcgcc acaataacaa acaattgcgt tttattatta    3960
   caaatccaat tttaaaaaaa gcggcagaac cggtcaaacc taaaagactg attacataaa    4020
   tcttattcaa atttcaaaag tgccccaggg gctagtatct acgacacacc gagcggcgaa    4080
   ctaataacgc tcactgaagg gaactccggt tccccgccgg cgcgcatggg tgagattcct    4140
   tgaagttgag tattggccgt ccgctctacc gaaagttacg ggcaccattc aacccggtcc    4200
   agcacggcgg ccgggtaacc gacttgctgc cccgagaatt atgcagcatt tttttggtgt    4260
   atgtgggccc caaatgaagt gcaggtcaaa ccttgacagt gacgacaaat cgttgggcgg    4320
   gtccagggcg aattttgcga caacatgtcg aggctcagca ggacctgcag gcatgcaagc    4380
   ttggcactgg ccgtcgtttt acaacgtcgt gactgggaaa accctggcgt tacccaactt    4440
   aatcgccttg cagcacatcc ccctttc                                         4467
<210> 8
   <211> 5431
   <212> DNA
   <213> artificial sequence
<220>
   <223> silencing cassette for gene TaCKX1
<400> 8
   aatcccacca aaacctgaac ctagcagttc agttgctcct ctcagagacg aatcgggtat 60
   tcaacaccct cataccaact actacgtcgt gtataacgga cctcatgccg gtatatacga 120
   tgactggggt tgtacaaagg cagcaacaaa cggtgttccc ggagttgcgc ataagaagtt 180
   tgccactatt acagaggcaa gagcagcagc tgacgcgtat acaacaagtc agcaaacaga 240
   taggttgaac ttcatcccca aaggagaagc tcaactcaag cccaagagct ttgcgaaggc 300
   cctaacaagc ccaccaaagc aaaaagccca ctgctcacgc taggaaccaa aaggcccagc 360
   agtgatccag ccccaaaaga gatctccttt gccccggaga ttacaatgga cgatttcctc 420
   tatctttacg atctaggaag gaagttcgaa ggtgaaggtg acgacactat gttcaccact 480
   gataatgaga aggttagcct cttcaatttc agaaagaatg ctgacccaca gatggttaga 540
   gaggcctacg cagcaggtct catcaagacg atctacccga gtaacaatct ccaggagatc 600
   aaataccttc ccaagaaggt taaagatgca gtcaaaagat tcaggactaa ttgcatcaag 660
   aacacagaga aagacatatt tctcaagatc agaagtacta ttccagtatg gacgattcaa 720
   ggcttgcttc ataaaccaag gcaagtaata gagattggag tctctaaaaa ggtagttcct 780
   actgaatcta aggccatgca tggagtctaa gattcaaatc gaggatctaa cagaactcgc 840
   cgtgaagact ggcgaacagt tcatacagag tcttttacga ctcaatgaca agaagaaaat 900
   cttcgtcaac atggtggagc acgacactct ggtctactcc aaaaatgtca aagatacagt 960
   ctcagaagac caaagggcta ttgagacttt tcaacaaagg ataatttcgg gaaacctcct 1020
   cggattccat tgcccagcta tctgtcactt catcgaaagg acagtagaaa aggaaggtgg 1080
   ctcctacaaa tgccatcatt gcgataaagg aaaggctatc attcaagatc tgcctctgcc 1140
   gacagtggtc ccaaagatgg acccccaccc acgaggagca tcgtggaaaa agaagacgtt 1200
   ccaaccacgt cttcaaagca agtggattga tgtgacatct ccactgacgt aagggatgac 1260
   gcacaatccc actatccttc gcaagaccct tcctctatat aaggaagttc atttcatttg 1320
   gagaggacac gctcgagccc ccatttaaat cccccctcga ggatcaagtg caaaggtccg 1380
   ccttgtttct cctctgtctc ttgatctgac taatcttggt ttatgattcg ttgagtaatt 1440
   ttggggaaag ctagcttcgt ccacagtttt tttttcgatg aacagtgccg cagtggcgct 1500
   gatcttgtat gctatcctgc aatcgtggtg aacttatgtc ttttatatcc ttcactacca 1560
   tgaaaagact agctagtaat ctttctcgat gtaacatcgt ccagcactgc tattaccgtg 1620
   tggtccatcc gacagtctgg ctgaacacat catacgatat tgagcaaaga tcgatctatc 1680
   ttccctgttc tttaatgaaa gacgtcattt tcatcagtat gatctaagaa tgttgcaact 1740
   tgcaaggagg cgtttctttc tttgaattta actaactcgt tgagtggccc tgtttctcgg 1800
   acgtaaggcc tttgctgctc cacacatgtc cattcgaatt ttaccgtgtt tagcaagggc 1860
   gaaaagtttg catcttgatg atttagcttg actatgcgat tgctttcctg gacccgtgca 1920
   gctgcggcca tgggagggcg cgccagagat atcagacgga ccgggccgcc gggtgaggaa 1980
   ctcgggcggg ttcttcaccg acgccgacgt cgccaggatc gtggccgtcg ccgcggcgag 2040
   gaacgctacc accgtgtacg tcatcgagac gacgctcaac tacgacagcg ccacggccgc 2100
   gtccgtggac caggagctca gctcggtgct ggcgacgctg aggcacgagg aggggcacgc 2160
   gttcgtgcgg gacgcgtcgt acctggagtt cctggaccgg gtgcacggcg aggagggggc 2220
   gctggacaag atcgggctgt ggcgtgtccc gcacccttgg ctcatcgtgc tcgtgccccg 2280
   ctcccgcatc gccgacttcg accgcggcgt cttcaagggc atcctccagg gcaccgacat 2340
   cgccgggcct ctcgtcgtct acccgctcaa caaatccaag tacgcactac gcgtccatcg 2400
   tgcatgcatg catgcgatcc tgcatggtat gtatcacgca cttaactgac actggctcgg 2460
   tcgtggtttc aggtgggacg acggcatgtc ggcggtgacg ccagcggagg aggtgttcta 2520
   cgcggtgtcg ctgcccttct cgtcggtggc caacgacctg aagcggctgg aggcgcagaa 2580
   ccagaagata ctgcggttct gcgacctcgc cggggtaggg tacaaggagt acctggcgca 2640
   ctacacggcc cacggcgact gggtccggca cttcggcggc aagtggcagc gcttcgtgga 2700
   gatgaaggac aagtacgacc ccaagaggct gctctcccca ggccaggaca tccctaggaa 2760
   ggtatacata tatgtttata attctttgtt tcccctctta ttcagatcga tcacatgcat 2820
   ctttcattgc tcgtttttcc ttacaagtag tctcatacat gctaatttct gtaaggtgtt 2880
   gggctggaaa ttaattaatt aattaattga cttgccaaga tccatatata tgtcctgata 2940
   ttaaatcttc gttcgttatg tttggttagg ctgatcaatg ttattctaga gtctagagaa 3000
   acacacccag gggttttcca actagctcca caagatggtg ggctagctga cctagatttg 3060
   aagtctcact ccttataatt attttatatt agatcatttt ctaatattcg tgtctttttt 3120
   tattctagag tctagatctt gtgttcaact ctcgttaaat catgtctctc gccactggag 3180
   aaacagatca ggagggttta ttttgggtat aggtcaaagc taagattgaa attcacaaat 3240
   agtaaaatca gaatccaacc aattttagta gccgagttgg tcaaaggaaa atgtatatag 3300
   ctagatttat tgttttggca aaaaaaaatc tgaatatgca aaatacttgt atatctttgt 3360
   attaagaaga tgaaaataag tagcagaaaa ttaaaaaatg gattatattt cctgggctaa 3420
   aagaattgtt gatttggcac aattaaattc agtgtcaagg ttttgtgcaa gaattcagtg 3480
   tgaaggaata gattctcttc aaaacaattt aatcattcat ctgatctgct caaagctctg 3540
   tgcatctccg ggtgcaacgg ccaggatatt tattgtgcag taaaaaaatg tcatatcccc 3600
   tagccaccca agaaactgct ccttaagtcc ttataagcac atatggcatt gtaatatata 3660
   tgtttgagtt ttagcgacaa tttttttaaa aacttttggt cctttttatg aacgttttaa 3720
   gtttcactgt cttttttttt cgaattttaa atgtagcttc aaattctaat ccccaatcca 3780
   aattgtaata aacttcaatt ctcctaatta acatcttaat tcatttattt gaaaaccagt 3840
   tcaaattctt ttaggctcac caaaccttaa acaattcaat tcagtggagc tcagagcgat 3900
   cgcagacccg gggatgtcct ggcctgggga gagcagcctc ttggggtcgt acttgtcctt 3960
   catctccacg aagcgctgcc acttgccgcc gaagtgccgg acccagtcgc cgtgggccgt   4020
   gtagtgcgcc aggtactcct tgtaccctac cccggcgagg tcgcagaacc gcagtatctt   4080
   ctggttctgc gcctccagcc gcttcaggtc gttggccacc gacgagaagg gcagcgacac   4140
   cgcgtagaac acctcctccg ctggcgtcac cgccgacatg ccgtcgtccc acctgaaacc   4200
   acgaccgagc cagtgtcagt taagtgcgtg atacatacca tgcaggatcg catgcatgca   4260
   tgcacgatgg acgcgtagtg cgtacttgga tttgttgagc gggtagacga cgagaggccc   4320
   ggcgatgtcg gtgccctgga ggatgccctt gaagacgccg cggtcgaagt cggcgatgcg   4380
   ggagcggggc acgagcacga tgagccaagg gtgcgggaca cgccacagcc cgatcttgtc   4440
   cagcgccccc tcctcgccgt gcacccggtc caggaactcc aggtacgacg cgtcccgcac   4500
   gaacgcgtgc ccctcctcgt gcctcagcgt cgccagcacc gagctgagct cctggtccac   4560
   ggacgcggcc gtggcgctgt cgtagttgag cgtcgtctcg atgacgtaca cggtggtagc   4620
   gttcctcgcc gcggcgacgg ccacgatcct ggcgacgtcg gcgtcggtga agaacccgcc   4680
   cgagttcctc acccggcggc cactagtggg tctagagtcc tgctttaatg agatatgcga   4740
   gacgcctatg atcgcatgat atttgctttc aattctgttg tgcacgttgt aaaaaacctg   4800
   agcatgtgta gctcagatcc ttaccgccgg tttcggttca ttctaatgaa tatatcaccc   4860
   gttactatcg tatttttatg aataatattc tccgttcaat ttactgattg taccctacta   4920
   cttatatgta caatattaaa atgaaaacaa tatattgtgc tgaataggtt tatagcgaca   4980
   tctatgatag agcgccacaa taacaaacaa ttgcgtttta ttattacaaa tccaatttta   5040
   aaaaaagcgg cagaaccggt caaacctaaa agactgatta cataaatctt attcaaattt   5100
   caaaagtgcc ccaggggcta gtatctacga cacaccgagc ggcgaactaa taacgctcac   5160
   tgaagggaac tccggttccc cgccggcgcg catgggtgag attccttgaa gttgagtatt   5220
   ggccgtccgc tctaccgaaa gttacgggca ccattcaacc cggtccagca cggcggccgg   5280
   gtaaccgact tgctgccccg agaattatgc agcatttttt tggtgtatgt gggccccaaa   5340
   tgaagtgcag gtcaaacctt gacagtgacg acaaatcgtt gggcgggtcc agggcgaatt   5400
   ttgcgacaac atgtcgaggc tcagcaggac c                                  5431
<210> 9
   <211> 8172
   <212> DNA
   <213> artificial sequence
<220>
   <223> T-DNA region of vector pMCG/HvCKX1
<400> 9
   aattctatgt ttgacagctt atcatcggat ctagtaacat agatgacacc gcgcgcgata    60
   atttatccta gtttgcgcgc tatattttgt tttctatcgc gtattaaatg tataattgcg   120
   ggactctaat cataaaaacc catctcataa ataacgtcat gcattacatg ttaattatta   180
   catgcttaac gtaattcaac agaaattata tgataatcat cgcaagaccg gcaacaggat   240
   tcaatcttaa gaaactttat tgccaaatgt tgaacgatct gcaggtcgac ggatcagatc   300
   tcggtgacgg gcaggaccgg acggggcggt accggcaggc tgaagtccag ctgccagaaa   360
   cccacgtcat gccagttccc gtgcttgaag ccggccgccc gcagcatgcc gcggggggca   420
   tatccgagcg cctcgtgcat gcgcacgctc gggtcgttgg gcagcccgat gacagcgacc   480
   acgctcttga agccctgtgc ctccagggac ttcagcaggt gggtgtagag cgtggagccc   540
   agtcccgtcc gctggtggcg gggggagacg tacacggtcg actcggccgt ccagtcgtag   600
   gcgttgcgtg ccttccaggg gcccgcgtag gcgatgccgg cgacctcgcc gtccacctcg   660
   gcgacgagcc agggatagcg ctcccgcaga cggacgaggt cgtccgtcca ctcctgcggt   720
   tcctgcggct cggtacggaa gttgaccgtg cttgtctcga tgtagtggtt gacgatggtg   780
   cagaccgccg gcatgtccgc ctcggtggca cggcggatgt cggccgggcg tcgttctggg   840
   ctcatggtta cttcctaatc gatggatcct ctagtgcaga agtaacacca aacaacaggg   900
   tgagcatcga caaaagaaac agtaccaagc aaataaatag cgtatgaagg cagggctaaa   960
   aaaatccaca tatagctgct gcatatgcca tcatccaagt atatcaagat caaaataatt  1020
   ataaaacata cttgtttatt ataatagata ggtactcaag gttagagcat atgaatagat  1080
   gctgcatatg ccatcatgta tatgcatcag taaaacccac atcaacatgt atacctatcc  1140
   tagatcgata tttccatcca tcttaaactc gtaactatga agatgtatga cacacacata  1200
   cagttccaaa attaataaat acaccaggta gtttgaaaca gtattctact ccgatctaga  1260
   acgaatgaac gaccgcccaa ccacaccaca tcatcacaac caagcgaaca aaaagcatct  1320
   ctgtatatgc atcagtaaaa cccgcatcaa catgtatacc tatcctagat cgatatttcc  1380
   atccatcatc ttcaattcgt aactatgaat atgtatggca cacacataca gatccaaaat  1440
   taataaatcc accaggtagt ttgaaacaga attctactcc gatctagaac gaccgcccaa  1500
   ccagaccaca tcatcacaac caagacaaaa aaaagcatga aaagatgacc cgacaaacaa  1560
   gtgcacggca tatattgaaa taaaggaaaa gggcaaacca aaccctatgc aacgaaacaa  1620
   aaaaaatcat gaaatcgatc ccgtctgcgg aacggctaga gccatcccag gattccccaa  1680
   agagaaacac tggcaagtta gcaatcagaa cgtgtctgac gtacaggtcg catccgtgta  1740
   cgaacgctag cagcacggat ctaacacaaa cacggatcta acacaaacat gaacagaagt  1800
   agaactaccg ggccctaacc atggaccgga acgccgatct agagaaggta gagagggggg  1860
   gggggggagg acgagcggcg taccttgaag cggaggtgcc gacgggtgga tttgggggag  1920
   atctggttgt gtgtgtgtgc gctccgaaca acacgaggtt ggggaaagag ggtgtggagg  1980
   gggtgtctat ttattacggc gggcgaggaa gggaaagcga aggagcggtg ggaaaggaat  2040
   cccccgtagc tgccgtgccg tgagaggagg aggaggccgc ctgccgtgcc ggctcacgtc 2100
   tgccgctccg ccacgcaatt tctggatgcc gacagcggag caagtccaac ggtggagcgg 2160
   aactctcgag aggggtccag aggcagcgac agagatgccg tgccgtctgc ttcgcttggc 2220
   ccgacgcgac gctgctggtt cgctggttgg tgtccgttag actcgtcgac ggcgtttaac 2280
   aggctggcat tatctactcg aaacaagaaa aatgtttcct tagttttttt aatttcttaa 2340
   agggtatttg tttaattttt agtcacttta ttttattcta ttttatatct aaattattaa 2400
   ataaaaaaac taaaatagag ttttagtttt cttaatttag aggctaaaat agaataaaat 2460
   agatgtacta aaaaaattag tctataaaaa ccattaaccc taaaccctaa atggatgtac 2520
   taataaaatg gatgaagtat tatataggtg aagctatttg caaaaaaaaa ggagaacaca 2580
   tgcacactaa aaagataaaa ctgtagagtc ctgttgtcaa aatactcaat tgtcctttag 2640
   accatgtcta actgttcatt tatatgattc tctaaaacac tgatattatt gtagtactat 2700
   agattatatt attcgtagag taaagtttaa atatatgtat aaagatagat aaactgcact 2760
   tcaaacaagt gtgacaaaaa aaatatgtgg taatttttta taacttagac atgcaatgct 2820
   cattatctct agagaggggc acgaccgggt cacgctgcac tgcactagag tcgaccctag 2880
   tgttcgagta ttatggcatt gggaaaactg tttttcttgt accatttgtt gtgcttgtaa 2940
   tttactgtgt tttttattcg gttttcgcta tcgaactgtg aaatggaaat ggatggagaa 3000
   gagttaatga atgatatggt ccttttgttc attctcaaat taatattatt tgttttttct 3060
   cttatttgtt gtgtgttgaa tttgaaatta taagagatat gcaaacattt tgttttgagt 3120
   aaaaatgtgt caaatcgtgg cctctaatga ccgaagttaa tatgaggagt aaaacacttg 3180
   tagttgtacc attatgctta ttcactaggc aacaaatata ttttcagacc tagaaaagct 3240
   gcaaatgtta ctgaatacaa gtatgtcctc ttgtgtttta gacatttatg aactttcctt 3300
   tatgtaattt tccagaatcc ttgtcagatt ctaatcattg ctttataatt atagttatac 3360
   tcatggattt gtagttgagt atgaaaatat tttttaatgc attttatgac ttgccaattg 3420
   attgacaaca tgcatcaatc tagggcatgc gggggttaat taacccccga attccaatcc 3480
   caccaaaacc tgaacctagc agttcagttg ctcctctcag agacgaatcg ggtattcaac 3540
   accctcatac caactactac gtcgtgtata acggacctca tgccggtata tacgatgact 3600
   ggggttgtac aaaggcagca acaaacggtg ttcccggagt tgcgcataag aagtttgcca 3660
   ctattacaga ggcaagagca gcagctgacg cgtatacaac aagtcagcaa acagataggt 3720
   tgaacttcat ccccaaagga gaagctcaac tcaagcccaa gagctttgcg aaggccctaa 3780
   caagcccacc aaagcaaaaa gcccactgct cacgctagga accaaaaggc ccagcagtga 3840
   tccagcccca aaagagatct cctttgcccc ggagattaca atggacgatt tcctctatct 3900
   ttacgatcta ggaaggaagt tcgaaggtga aggtgacgac actatgttca ccactgataa 3960
   tgagaaggtt agcctcttca atttcagaaa gaatgctgac ccacagatgg ttagagaggc 4020
   ctacgcagca ggtctcatca agacgatcta cccgagtaac aatctccagg agatcaaata 4080
   ccttcccaag aaggttaaag atgcagtcaa aagattcagg actaattgca tcaagaacac 4140
   agagaaagac atatttctca agatcagaag tactattcca gtatggacga ttcaaggctt 4200
   gcttcataaa ccaaggcaag taatagagat tggagtctct aaaaaggtag ttcctactga 4260
   atctaaggcc atgcatggag tctaagattc aaatcgagga tctaacagaa ctcgccgtga 4320
   agactggcga acagttcata cagagtcttt tacgactcaa tgacaagaag aaaatcttcg 4380
   tcaacatggt ggagcacgac actctggtct actccaaaaa tgtcaaagat acagtctcag 4440
   aagaccaaag ggctattgag acttttcaac aaaggataat ttcgggaaac ctcctcggat 4500
   tccattgccc agctatctgt cacttcatcg aaaggacagt agaaaaggaa ggtggctcct 4560
   acaaatgcca tcattgcgat aaaggaaagg ctatcattca agatctgcct ctgccgacag 4620
   tggtcccaaa gatggacccc cacccacgag gagcatcgtg gaaaaagaag acgttccaac 4680
   cacgtcttca aagcaagtgg attgatgtga catctccact gacgtaaggg atgacgcaca 4740
   atcccactat ccttcgcaag acccttcctc tatataagga agttcatttc atttggagag 4800
   gacacgctcg agcccccatt taaatccccc ctcgaggatc aagtgcaaag gtccgccttg 4860
   tttctcctct gtctcttgat ctgactaatc ttggtttatg attcgttgag taattttggg 4920
   gaaagctagc ttcgtccaca gttttttttt cgatgaacag tgccgcagtg gcgctgatct 4980
   tgtatgctat cctgcaatcg tggtgaactt atgtctttta tatccttcac taccatgaaa 5040
   agactagcta gtaatctttc tcgatgtaac atcgtccagc actgctatta ccgtgtggtc 5100
   catccgacag tctggctgaa cacatcatac gatattgagc aaagatcgat ctatcttccc 5160
   tgttctttaa tgaaagacgt cattttcatc agtatgatct aagaatgttg caacttgcaa 5220
   ggaggcgttt ctttctttga atttaactaa ctcgttgagt ggccctgttt ctcggacgta 5280
   aggcctttgc tgctccacac atgtccattc gaattttacc gtgtttagca agggcgaaaa 5340
   gtttgcatct tgatgattta gcttgactat gcgattgctt tcctggaccc gtgcagctgc 5400
   ggccatggga gggcgcgcca gagatatcag acggaccgcc tcatccctgg ctcaacgtgc 5460
   tcgtgccccg ctccggcatc gccgacttcg accgcgccgt cttcaggggc atcctccagg 5520
   gcaccgacat cgccgggccc ctcgtcgtct acccactcaa caaatccaag tgggacgacg 5580
   gcatgtcggc ggtgacgccg gcggaggagg tgttctacgc ggtgtcgatg ctcttctcgt 5640
   cggtggccaa cgacctgagg cggctggagg cgcagaacca gaagatactg cggttctgcg 5700
   acctcgccgg gatagggtac aaggagtacc tggcgcacta cacggcccac ggcgactggg 5760
   tccggcactt cggcggcaag tggaagcact tcgtggagat gaaggacaag tacgacccca 5820
   agaagctgct ctccccgggc caagacatct tcaactaaag acctaggaag gtatacatat 5880
   atgtttataa ttctttgttt cccctcttat tcagatcgat cacatgcatc tttcattgct 5940
   cgtttttcct tacaagtagt ctcatacatg ctaatttctg taaggtgttg ggctggaaat 6000
   taattaatta attaattgac ttgccaagat ccatatatat gtcctgatat taaatcttcg 6060
   ttcgttatgt ttggttaggc tgatcaatgt tattctagag tctagagaaa cacacccagg    6120
   ggttttccaa ctagctccac aagatggtgg gctagctgac ctagatttga agtctcactc    6180
   cttataatta ttttatatta gatcattttc taatattcgt gtcttttttt attctagagt    6240
   ctagatcttg tgttcaactc tcgttaaatc atgtctctcg ccactggaga aacagatcag    6300
   gagggtttat tttgggtata ggtcaaagct aagattgaaa ttcacaaata gtaaaatcag    6360
   aatccaacca attttagtag ccgagttggt caaaggaaaa tgtatatagc tagatttatt    6420
   gttttggcaa aaaaaaatct gaatatgcaa aatacttgta tatctttgta ttaagaagat    6480
   gaaaataagt agcagaaaat taaaaaatgg attatatttc ctgggctaaa agaattgttg    6540
   atttggcaca attaaattca gtgtcaaggt tttgtgcaag aattcagtgt gaaggaatag    6600
   attctcttca aaacaattta atcattcatc tgatctgctc aaagctctgt gcatctccgg    6660
   gtgcaacggc caggatattt attgtgcagt aaaaaaatgt catatcccct agccacccaa    6720
   gaaactgctc cttaagtcct tataagcaca tatggcattg taatatatat gtttgagttt    6780
   tagcgacaat tttttaaaaa acttttggtc ctttttatga acgttttaag tttcactgtc    6840
   tttttttttc gaattttaaa tgtagcttca aattctaatc cccaatccaa attgtaataa    6900
   acttcaattc tcctaattaa catcttaatt catttatttg aaaaccagtt caaattcttt    6960
   taggctcacc aaaccttaaa caattcaatt cagtggagct cagagcgatc gcagacccgg    7020
   gaggttagtt gaagatgtct tggcccgggg agagcagctt cttggggtcg tacttgtcct    7080
   tcatctccac gaagtgcttc cacttgccgc cgaagtgccg gacccagtcg ccgtgggccg    7140
   tgtagtgcgc caggtactcc ttgtacccta tcccggcgag gtcgcagaac cgcagtatct    7200
   tctggttctg cgcctccagc cgcctcaggt cgttggccac cgacgagaag agcatcgaca    7260
   ccgcgtagaa cacctcctcc gccggcgtca ccgccgacat gccgtcgtcc cacttggatt    7320
   tgttgagtgg gtagacgacg aggggcccgg cgatgtcggt gccctggagg atgcccctga    7380
   agacggcgcg gtcgaagtcg gcgatgccgg agcggggcac gagcacgttg agccagggat    7440
   gaggactagt gggtctagag tcctgcttta atgagatatg cgagacgcct atgatcgcat    7500
   gatatttgct ttcaattctg ttgtgcacgt tgtaaaaaac ctgagcatgt gtagctcaga    7560
   tccttaccgc cggtttcggt tcattctaat gaatatatca cccgttacta tcgtattttt    7620
   atgaataata ttctccgttc aatttactga ttgtacccta ctacttatat gtacaatatt    7680
   aaaatgaaaa caatatattg tgctgaatag gtttatagcg acatctatga tagagcgcca    7740
   caataacaaa caattgcgtt ttattattac aaatccaatt ttaaaaaaag cggcagaacc    7800
   ggtcaaacct aaaagactga ttacataaat cttattcaaa tttcaaaagt gccccagggg    7860
   ctagtatcta cgacacaccg agcggcgaac taataacgct cactgaaggg aactccggtt    7920
   ccccgccggc gcgcatgggt gagattcctt gaagttgagt attggccgtc cgctctaccg    7980
   aaagttacgg gcaccattca acccggtcca gcacggcggc cgggtaaccg acttgctgcc    8040
   ccgagaatta tgcagcattt ttttggtgta tgtgggcccc aaatgaagtg caggtcaaac    8100
   cttgacagtg acgacaaatc gttgggcggg tccagggcga attttgcgac aacatgtcga    8160
   ggctcagcag ga                                                        8172
<210> 10
   <211> 414
   <212> DNA
   <213> artificial sequence
<220>
   <223> sequence of the inserted HvCKX1 gene fragment, sense orientation
<400> 10
   atccctggct caacgtgctc gtgccccgct ccggcatcgc cgacttcgac cgcgccgtct      60
   tcaggggcat cctccagggc accgacatcg ccgggcccct cgtcgtctac ccactcaaca     120
   aatccaagtg ggacgacggc atgtcggcgg tgacgccggc ggaggaggtg ttctacgcgg     180
   tgtcgatgct cttctcgtcg gtggccaacg acctgaggcg gctggaggcg cagaaccaga     240
   agatactgcg gttctgcgac ctcgccggga tagggtacaa ggagtacctg gcgcactaca     300
   cggcccacgg cgactgggtc cggcacttcg gcggcaagtg gaagcacttc gtggagatga     360
   aggacaagta cgaccccaag aagctgctct ccccgggcca agacatcttc aact           414
<210> 11
   <211> 7942
   <212> DNA
   <213> artificial sequence
<220>
   <223> T-DNA region of vector pMCG/HvCKX2
<400> 11
   aattctatgt ttgacagctt atcatcggat ctagtaacat agatgacacc gcgcgcgata      60
   atttatccta gtttgcgcgc tatattttgt tttctatcgc gtattaaatg tataattgcg     120
   ggactctaat cataaaaacc catctcataa ataacgtcat gcattacatg ttaattatta     180
   catgcttaac gtaattcaac agaaattata tgataatcat cgcaagaccg gcaacaggat     240
   tcaatcttaa gaaactttat tgccaaatgt tgaacgatct gcaggtcgac ggatcagatc     300
   tcggtgacgg gcaggaccgg acggggcggt accggcaggc tgaagtccag ctgccagaaa     360
   cccacgtcat gccagttccc gtgcttgaag ccggccgccc gcagcatgcc gcggggggca     420
   tatccgagcg cctcgtgcat gcgcacgctc gggtcgttgg gcagcccgat gacagcgacc     480
   acgctcttga agccctgtgc ctccagggac ttcagcaggt gggtgtagag cgtggagccc      540
   agtcccgtcc gctggtggcg gggggagacg tacacggtcg actcggccgt ccagtcgtag      600
   gcgttgcgtg ccttccaggg gcccgcgtag gcgatgccgg cgacctcgcc gtccacctcg      660
   gcgacgagcc agggatagcg ctcccgcaga cggacgaggt cgtccgtcca ctcctgcggt      720
   tcctgcggct cggtacggaa gttgaccgtg cttgtctcga tgtagtggtt gacgatggtg      780
   cagaccgccg gcatgtccgc ctcggtggca cggcggatgt cggccgggcg tcgttctggg      840
   ctcatggtta cttcctaatc gatggatcct ctagtgcaga agtaacacca aacaacaggg      900
   tgagcatcga caaaagaaac agtaccaagc aaataaatag cgtatgaagg cagggctaaa      960
   aaaatccaca tatagctgct gcatatgcca tcatccaagt atatcaagat caaaataatt     1020
   ataaaacata cttgtttatt ataatagata ggtactcaag gttagagcat atgaatagat     1080
   gctgcatatg ccatcatgta tatgcatcag taaaacccac atcaacatgt atacctatcc     1140
   tagatcgata tttccatcca tcttaaactc gtaactatga agatgtatga cacacacata     1200
   cagttccaaa attaataaat acaccaggta gtttgaaaca gtattctact ccgatctaga     1260
   acgaatgaac gaccgcccaa ccacaccaca tcatcacaac caagcgaaca aaaagcatct     1320
   ctgtatatgc atcagtaaaa cccgcatcaa catgtatacc tatcctagat cgatatttcc     1380
   atccatcatc ttcaattcgt aactatgaat atgtatggca cacacataca gatccaaaat     1440
   taataaatcc accaggtagt ttgaaacaga attctactcc gatctagaac gaccgcccaa     1500
   ccagaccaca tcatcacaac caagacaaaa aaaagcatga aaagatgacc cgacaaacaa     1560
   gtgcacggca tatattgaaa taaaggaaaa gggcaaacca aaccctatgc aacgaaacaa     1620
   aaaaaatcat gaaatcgatc ccgtctgcgg aacggctaga gccatcccag gattccccaa     1680
   agagaaacac tggcaagtta gcaatcagaa cgtgtctgac gtacaggtcg catccgtgta     1740
   cgaacgctag cagcacggat ctaacacaaa cacggatcta acacaaacat gaacagaagt     1800
   agaactaccg ggccctaacc atggaccgga acgccgatct agagaaggta gagagggggg     1860
   gggggggagg acgagcggcg taccttgaag cggaggtgcc gacgggtgga tttgggggag     1920
   atctggttgt gtgtgtgtgc gctccgaaca acacgaggtt ggggaaagag ggtgtggagg     1980
   gggtgtctat ttattacggc gggcgaggaa gggaaagcga aggagcggtg ggaaaggaat     2040
   cccccgtagc tgccgtgccg tgagaggagg aggaggccgc ctgccgtgcc ggctcacgtc     2100
   tgccgctccg ccacgcaatt tctggatgcc gacagcggag caagtccaac ggtggagcgg     2160
   aactctcgag aggggtccag aggcagcgac agagatgccg tgccgtctgc ttcgcttggc     2220
   ccgacgcgac gctgctggtt cgctggttgg tgtccgttag actcgtcgac ggcgtttaac     2280
   aggctggcat tatctactcg aaacaagaaa aatgtttcct tagttttttt aatttcttaa     2340
   agggtatttg tttaattttt agtcacttta ttttattcta ttttatatct aaattattaa     2400
   ataaaaaaac taaaatagag ttttagtttt cttaatttag aggctaaaat agaataaaat     2460
   agatgtacta aaaaaattag tctataaaaa ccattaaccc taaaccctaa atggatgtac     2520
   taataaaatg gatgaagtat tatataggtg aagctatttg caaaaaaaaa ggagaacaca     2580
   tgcacactaa aaagataaaa ctgtagagtc ctgttgtcaa aatactcaat tgtcctttag     2640
   accatgtcta actgttcatt tatatgattc tctaaaacac tgatattatt gtagtactat     2700
   agattatatt attcgtagag taaagtttaa atatatgtat aaagatagat aaactgcact     2760
   tcaaacaagt gtgacaaaaa aaatatgtgg taatttttta taacttagac atgcaatgct      2820
   cattatctct agagaggggc acgaccgggt cacgctgcac tgcactagag tcgaccctag     2880
   tgttcgagta ttatggcatt gggaaaactg tttttcttgt accatttgtt gtgcttgtaa     2940
   tttactgtgt tttttattcg gttttcgcta tcgaactgtg aaatggaaat ggatggagaa     3000
   gagttaatga atgatatggt ccttttgttc attctcaaat taatattatt tgttttttct      3060
   cttatttgtt gtgtgttgaa tttgaaatta taagagatat gcaaacattt tgttttgagt     3120
   aaaaatgtgt caaatcgtgg cctctaatga ccgaagttaa tatgaggagt aaaacacttg     3180
   tagttgtacc attatgctta ttcactaggc aacaaatata ttttcagacc tagaaaagct     3240
   gcaaatgtta ctgaatacaa gtatgtcctc ttgtgtttta gacatttatg aactttcctt     3300
   tatgtaattt tccagaatcc ttgtcagatt ctaatcattg ctttataatt atagttatac     3360
   tcatggattt gtagttgagt atgaaaatat tttttaatgc attttatgac ttgccaattg     3420
   attgacaaca tgcatcaatc tagggcatgc gggggttaat taacccccga attccaatcc      3480
   caccaaaacc tgaacctagc agttcagttg ctcctctcag agacgaatcg ggtattcaac     3540
   accctcatac caactactac gtcgtgtata acggacctca tgccggtata tacgatgact     3600
   ggggttgtac aaaggcagca acaaacggtg ttcccggagt tgcgcataag aagtttgcca     3660
   ctattacaga ggcaagagca gcagctgacg cgtatacaac aagtcagcaa acagataggt     3720
   tgaacttcat ccccaaagga gaagctcaac tcaagcccaa gagctttgcg aaggccctaa     3780
   caagcccacc aaagcaaaaa gcccactgct cacgctagga accaaaaggc ccagcagtga     3840
   tccagcccca aaagagatct cctttgcccc ggagattaca atggacgatt tcctctatct     3900
   ttacgatcta ggaaggaagt tcgaaggtga aggtgacgac actatgttca ccactgataa     3960
   tgagaaggtt agcctcttca atttcagaaa gaatgctgac ccacagatgg ttagagaggc     4020
   ctacgcagca ggtctcatca agacgatcta cccgagtaac aatctccagg agatcaaata     4080
   ccttcccaag aaggttaaag atgcagtcaa aagattcagg actaattgca tcaagaacac     4140
   agagaaagac atatttctca agatcagaag tactattcca gtatggacga ttcaaggctt     4200
   gcttcataaa ccaaggcaag taatagagat tggagtctct aaaaaggtag ttcctactga     4260
   atctaaggcc atgcatggag tctaagattc aaatcgagga tctaacagaa ctcgccgtga     4320
   agactggcga acagttcata cagagtcttt tacgactcaa tgacaagaag aaaatcttcg     4380
   tcaacatggt ggagcacgac actctggtct actccaaaaa tgtcaaagat acagtctcag     4440
   aagaccaaag ggctattgag acttttcaac aaaggataat ttcgggaaac ctcctcggat     4500
   tccattgccc agctatctgt cacttcatcg aaaggacagt agaaaaggaa ggtggctcct    4560
   acaaatgcca tcattgcgat aaaggaaagg ctatcattca agatctgcct ctgccgacag    4620
   tggtcccaaa gatggacccc cacccacgag gagcatcgtg gaaaaagaag acgttccaac    4680
   cacgtcttca aagcaagtgg attgatgtga catctccact gacgtaaggg atgacgcaca    4740
   atcccactat ccttcgcaag acccttcctc tatataagga agttcatttc atttggagag    4800
   gacacgctcg agcccccatt taaatccccc ctcgaggatc aagtgcaaag gtccgccttg    4860
   tttctcctct gtctcttgat ctgactaatc ttggtttatg attcgttgag taattttggg    4920
   gaaagctagc ttcgtccaca gttttttttt cgatgaacag tgccgcagtg gcgctgatct    4980
   tgtatgctat cctgcaatcg tggtgaactt atgtctttta tatccttcac taccatgaaa    5040
   agactagcta gtaatctttc tcgatgtaac atcgtccagc actgctatta ccgtgtggtc    5100
   catccgacag tctggctgaa cacatcatac gatattgagc aaagatcgat ctatcttccc    5160
   tgttctttaa tgaaagacgt cattttcatc agtatgatct aagaatgttg caacttgcaa    5220
   ggaggcgttt ctttctttga atttaactaa ctcgttgagt ggccctgttt ctcggacgta    5280
   aggcctttgc tgctccacac atgtccattc gaattttacc gtgtttagca agggcgaaaa    5340
   gtttgcatct tgatgattta gcttgactat gcgattgctt tcctggaccc gtgcagctgc    5400
   ggccatggga gggcgcgcca gagatatcag acggaccgac tagtgaggcg aactctggat    5460
   aaatgtcttg aataagacgt tgaagtatgg tttggcgccg aagtcatgga cagactacct    5520
   ccaccttacg gttgggggca cgttgtcaaa tgcgggcgtc agcgggcaga cattccggca    5580
   tggtccacag atcagcaatg tgaacgaatt ggagattgtg actggaagag gtgatattgt    5640
   cacttgctca ccagaacaga actctgatct cttccgtgct gctcttggtg gtctgggtca    5700
   gtttgagctc cctaggaagg tatacatata tgtttataat tctttgtttc ccctcttatt    5760
   cagatcgatc acatgcatct ttcattgctc gtttttcctt acaagtagtc tcatacatgc    5820
   taatttctgt aaggtgttgg gctggaaatt aattaattaa ttaattgact tgccaagatc    5880
   catatatatg tcctgatatt aaatcttcgt tcgttatgtt tggttaggct gatcaatgtt    5940
   attctagagt ctagagaaac acacccaggg gttttccaac tagctccaca agatggtggg    6000
   ctagctgacc tagatttgaa gtctcactcc ttataattat tttatattag atcattttct    6060
   aatattcgtg tcttttttta ttctagagtc tagatcttgt gttcaactct cgttaaatca    6120
   tgtctctcgc cactggagaa acagatcagg agggtttatt ttgggtatag gtcaaagcta    6180
   agattgaaat tcacaaatag taaaatcaga atccaaccaa ttttagtagc cgagttggtc    6240
   aaaggaaaat gtatatagct agatttattg ttttggcaaa aaaaaatctg aatatgcaaa    6300
   atacttgtat atctttgtat taagaagatg aaaataagta gcagaaaatt aaaaaatgga    6360
   ttatatttcc tgggctaaaa gaattgttga tttggcacaa ttaaattcag tgtcaaggtt    6420
   ttgtgcaaga attcagtgtg aaggaataga ttctcttcaa aacaatttaa tcattcatct    6480
   gatctgctca aagctctgtg catctccggg tgcaacggcc aggatattta ttgtgcagta    6540
   aaaaaatgtc atatccccta gccacccaag aaactgctcc ttaagtcctt ataagcacat    6600
   atggcattgt aatatatatg tttgagtttt agcgacaatt tttttaaaaa cttttggtcc    6660
   tttttatgaa cgttttaagt ttcactgtct ttttttttcg aattttaaat gtagcttcaa    6720
   attctaatcc ccaatccaaa ttgtaataaa cttcaattct cctaattaac atcttaattc    6780
   atttatttga aaaccagttc aaattctttt aggctcacca aaccttaaac aattcaattc    6840
   agtggagctc aaactgaccc agaccaccaa gagcagcacg gaagagatca gagttctgtt    6900
   ctggtgagca agtgacaata tcacctcttc cagtcacaat ctccaattcg ttcacattgc    6960
   tgatctgtgg accatgccgg aatgtctgcc cgctgacgcc cgcatttgac aacgtgcccc    7020
   caaccgtaag gtggaggtag tctgtccatg acttcggcgc caaaccatac ttcaacgtct    7080
   tattcaagac atttatccag agttcgcctc actagtgggt ctagagtcct gctttaatga    7140
   gatatgcgag acgcctatga tcgcatgata tttgctttca attctgttgt gcacgttgta    7200
   aaaaacctga gcatgtgtag ctcagatcct taccgccggt ttcggttcat tctaatgaat    7260
   atatcacccg ttactatcgt atttttatga ataatattct ccgttcaatt tactgattgt    7320
   accctactac ttatatgtac aatattaaaa tgaaaacaat atattgtgct gaataggttt    7380
   atagcgacat ctatgataga gcgccacaat aacaaacaat tgcgttttat tattacaaat    7440
   ccaattttaa aaaaagcggc agaaccggtc aaacctaaaa gactgattac ataaatctta    7500
   ttcaaatttc aaaagtgccc caggggctag tatctacgac acaccgagcg gcgaactaat    7560
   aacgctcact gaagggaact ccggttcccc gccggcgcgc atgggtgaga ttccttgaag    7620
   ttgagtattg gccgtccgct ctaccgaaag ttacgggcac cattcaaccc ggtccagcac    7680
   ggcggccggg taaccgactt gctgccccga gaattatgca gcattttttt ggtgtatgtg    7740
   ggccccaaat gaagtgcagg tcaaaccttg acagtgacga caaatcgttg ggcgggtcca    7800
   gggcgaattt tgcgacaaca tgtcgaggct cagcaggacc tgcaggcatg caagcttggc    7860
   actggccgtc gttttacaac gtcgtgactg ggaaaaccct ggcgttaccc aacttaatcg    7920
   ccttgcagca catccccctt tc                                             7942
<210> 12
   <211> 260
   <212> DNA
   <213> artificial sequence
<220>
   <223> sequence of the inserted HvCKX2 gene fragment, sense orientation
<400> 12
   gaggcgaact ctggataaat gtcttgaata agacgttgaa gtatggtttg gcgccgaagt        60
   catggacaga ctacctccac cttacggttg ggggcacgtt gtcaaatgcg ggcgtcagcg       120
   ggcagacatt ccggcatggt ccacagatca gcaatgtgaa cgaattggag attgtgactg       180
   gaagaggtga tattgtcact tgctcaccag aacagaactc tgatctcttc cgtgctgctc       240
   ttggtggtct gggtcagttt                                                   260
<210> 13
   <211> 8906
   <212> DNA
   <213> artificial sequence
<220>
   <223> T-DNA region of the vector pMCG/TaCKX1
<400> 13
   aattctatgt ttgacagctt atcatcggat ctagtaacat agatgacacc gcgcgcgata      60
   atttatccta gtttgcgcgc tatattttgt tttctatcgc gtattaaatg tataattgcg     120
   ggactctaat cataaaaacc catctcataa ataacgtcat gcattacatg ttaattatta     180
   catgcttaac gtaattcaac agaaattata tgataatcat cgcaagaccg gcaacaggat     240
   tcaatcttaa gaaactttat tgccaaatgt tgaacgatct gcaggtcgac ggatcagatc     300
   tcggtgacgg gcaggaccgg acggggcggt accggcaggc tgaagtccag ctgccagaaa     360
   cccacgtcat gccagttccc gtgcttgaag ccggccgccc gcagcatgcc gcggggggca     420
   tatccgagcg cctcgtgcat gcgcacgctc gggtcgttgg gcagcccgat gacagcgacc     480
   acgctcttga agccctgtgc ctccagggac ttcagcaggt gggtgtagag cgtggagccc     540
   agtcccgtcc gctggtggcg gggggagacg tacacggtcg actcggccgt ccagtcgtag     600
   gcgttgcgtg ccttccaggg gcccgcgtag gcgatgccgg cgacctcgcc gtccacctcg     660
   gcgacgagcc agggatagcg ctcccgcaga cggacgaggt cgtccgtcca ctcctgcggt     720
   tcctgcggct cggtacggaa gttgaccgtg cttgtctcga tgtagtggtt gacgatggtg     780
   cagaccgccg gcatgtccgc ctcggtggca cggcggatgt cggccgggcg tcgttctggg     840
   ctcatggtta cttcctaatc gatggatcct ctagtgcaga agtaacacca aacaacaggg     900
   tgagcatcga caaaagaaac agtaccaagc aaataaatag cgtatgaagg cagggctaaa     960
   aaaatccaca tatagctgct gcatatgcca tcatccaagt atatcaagat caaaataatt    1020
   ataaaacata cttgtttatt ataatagata ggtactcaag gttagagcat atgaatagat    1080
   gctgcatatg ccatcatgta tatgcatcag taaaacccac atcaacatgt atacctatcc    1140
   tagatcgata tttccatcca tcttaaactc gtaactatga agatgtatga cacacacata    1200
   cagttccaaa attaataaat acaccaggta gtttgaaaca gtattctact ccgatctaga    1260
   acgaatgaac gaccgcccaa ccacaccaca tcatcacaac caagcgaaca aaaagcatct    1320
   ctgtatatgc atcagtaaaa cccgcatcaa catgtatacc tatcctagat cgatatttcc    1380
   atccatcatc ttcaattcgt aactatgaat atgtatggca cacacataca gatccaaaat    1440
   taataaatcc accaggtagt ttgaaacaga attctactcc gatctagaac gaccgcccaa    1500
   ccagaccaca tcatcacaac caagacaaaa aaaagcatga aaagatgacc cgacaaacaa    1560
   gtgcacggca tatattgaaa taaaggaaaa gggcaaacca aaccctatgc aacgaaacaa    1620
   aaaaaatcat gaaatcgatc ccgtctgcgg aacggctaga gccatcccag gattccccaa    1680
   agagaaacac tggcaagtta gcaatcagaa cgtgtctgac gtacaggtcg catccgtgta    1740
   cgaacgctag cagcacggat ctaacacaaa cacggatcta acacaaacat gaacagaagt    1800
   agaactaccg ggccctaacc atggaccgga acgccgatct agagaaggta gagagggggg    1860
   gggggggagg acgagcggcg taccttgaag cggaggtgcc gacgggtgga tttgggggag    1920
   atctggttgt gtgtgtgtgc gctccgaaca acacgaggtt ggggaaagag ggtgtggagg    1980
   gggtgtctat ttattacggc gggcgaggaa gggaaagcga aggagcggtg ggaaaggaat    2040
   cccccgtagc tgccgtgccg tgagaggagg aggaggccgc ctgccgtgcc ggctcacgtc    2100
   tgccgctccg ccacgcaatt tctggatgcc gacagcggag caagtccaac ggtggagcgg    2160
   aactctcgag aggggtccag aggcagcgac agagatgccg tgccgtctgc ttcgcttggc    2220
   ccgacgcgac gctgctggtt cgctggttgg tgtccgttag actcgtcgac ggcgtttaac    2280
   aggctggcat tatctactcg aaacaagaaa aatgtttcct tagttttttt aatttcttaa    2340
   agggtatttg tttaattttt agtcacttta ttttattcta ttttatatct aaattattaa    2400
   ataaaaaaac taaaatagag ttttagtttt cttaatttag aggctaaaat agaataaaat    2460
   agatgtacta aaaaaattag tctataaaaa ccattaaccc taaaccctaa atggatgtac    2520
   taataaaatg gatgaagtat tatataggtg aagctatttg caaaaaaaaa ggagaacaca    2580
   tgcacactaa aaagataaaa ctgtagagtc ctgttgtcaa aatactcaat tgtcctttag    2640
   accatgtcta actgttcatt tatatgattc tctaaaacac tgatattatt gtagtactat    2700
   agattatatt attcgtagag taaagtttaa atatatgtat aaagatagat aaactgcact    2760
   tcaaacaagt gtgacaaaaa aaatatgtgg taatttttta taacttagac atgcaatgct    2820
   cattatctct agagaggggc acgaccgggt cacgctgcac tgcactagag tcgaccctag    2880
   tgttcgagta ttatggcatt gggaaaactg tttttcttgt accatttgtt gtgcttgtaa    2940
   tttactgtgt tttttattcg gttttcgcta tcgaactgtg aaatggaaat ggatggagaa    3000
   gagttaatga atgatatggt ccttttgttc attctcaaat taatattatt tgttttttct    3060
   cttatttgtt gtgtgttgaa tttgaaatta taagagatat gcaaacattt tgttttgagt    3120
   aaaaatgtgt caaatcgtgg cctctaatga ccgaagttaa tatgaggagt aaaacacttg    3180
   tagttgtacc attatgctta ttcactaggc aacaaatata ttttcagacc tagaaaagct    3240
   gcaaatgtta ctgaatacaa gtatgtcctc ttgtgtttta gacatttatg aactttcctt    3300
   tatgtaattt tccagaatcc ttgtcagatt ctaatcattg ctttataatt atagttatac 3360
   tcatggattt gtagttgagt atgaaaatat tttttaatgc attttatgac ttgccaattg 3420
   attgacaaca tgcatcaatc tagggcatgc gggggttaat taacccccga attccaatcc 3480
   caccaaaacc tgaacctagc agttcagttg ctcctctcag agacgaatcg ggtattcaac 3540
   accctcatac caactactac gtcgtgtata acggacctca tgccggtata tacgatgact 3600
   ggggttgtac aaaggcagca acaaacggtg ttcccggagt tgcgcataag aagtttgcca 3660
   ctattacaga ggcaagagca gcagctgacg cgtatacaac aagtcagcaa acagataggt 3720
   tgaacttcat ccccaaagga gaagctcaac tcaagcccaa gagctttgcg aaggccctaa 3780
   caagcccacc aaagcaaaaa gcccactgct cacgctagga accaaaaggc ccagcagtga 3840
   tccagcccca aaagagatct cctttgcccc ggagattaca atggacgatt tcctctatct 3900
   ttacgatcta ggaaggaagt tcgaaggtga aggtgacgac actatgttca ccactgataa 3960
   tgagaaggtt agcctcttca atttcagaaa gaatgctgac ccacagatgg ttagagaggc 4020
   ctacgcagca ggtctcatca agacgatcta cccgagtaac aatctccagg agatcaaata 4080
   ccttcccaag aaggttaaag atgcagtcaa aagattcagg actaattgca tcaagaacac 4140
   agagaaagac atatttctca agatcagaag tactattcca gtatggacga ttcaaggctt 4200
   gcttcataaa ccaaggcaag taatagagat tggagtctct aaaaaggtag ttcctactga 4260
   atctaaggcc atgcatggag tctaagattc aaatcgagga tctaacagaa ctcgccgtga 4320
   agactggcga acagttcata cagagtcttt tacgactcaa tgacaagaag aaaatcttcg 4380
   tcaacatggt ggagcacgac actctggtct actccaaaaa tgtcaaagat acagtctcag 4440
   aagaccaaag ggctattgag acttttcaac aaaggataat ttcgggaaac ctcctcggat 4500
   tccattgccc agctatctgt cacttcatcg aaaggacagt agaaaaggaa ggtggctcct 4560
   acaaatgcca tcattgcgat aaaggaaagg ctatcattca agatctgcct ctgccgacag 4620
   tggtcccaaa gatggacccc cacccacgag gagcatcgtg gaaaaagaag acgttccaac 4680
   cacgtcttca aagcaagtgg attgatgtga catctccact gacgtaaggg atgacgcaca 4740
   atcccactat ccttcgcaag acccttcctc tatataagga agttcatttc atttggagag 4800
   gacacgctcg agcccccatt taaatccccc ctcgaggatc aagtgcaaag gtccgccttg 4860
   tttctcctct gtctcttgat ctgactaatc ttggtttatg attcgttgag taattttggg 4920
   gaaagctagc ttcgtccaca gttttttttt cgatgaacag tgccgcagtg gcgctgatct 4980
   tgtatgctat cctgcaatcg tggtgaactt atgtctttta tatccttcac taccatgaaa 5040
   agactagcta gtaatctttc tcgatgtaac atcgtccagc actgctatta ccgtgtggtc 5100
   catccgacag tctggctgaa cacatcatac gatattgagc aaagatcgat ctatcttccc 5160
   tgttctttaa tgaaagacgt cattttcatc agtatgatct aagaatgttg caacttgcaa 5220
   ggaggcgttt ctttctttga atttaactaa ctcgttgagt ggccctgttt ctcggacgta 5280
   aggcctttgc tgctccacac atgtccattc gaattttacc gtgtttagca agggcgaaaa 5340
   gtttgcatct tgatgattta gcttgactat gcgattgctt tcctggaccc gtgcagctgc 5400
   ggccatggga gggcgcgcca gagatatcag acggaccggg ccgccgggtg aggaactcgg 5460
   gcgggttctt caccgacgcc gacgtcgcca ggatcgtggc cgtcgccgcg gcgaggaacg 5520
   ctaccaccgt gtacgtcatc gagacgacgc tcaactacga cagcgccacg gccgcgtccg 5580
   tggaccagga gctcagctcg gtgctggcga cgctgaggca cgaggagggg cacgcgttcg 5640
   tgcgggacgc gtcgtacctg gagttcctgg accgggtgca cggcgaggag ggggcgctgg 5700
   acaagatcgg gctgtggcgt gtcccgcacc cttggctcat cgtgctcgtg ccccgctccc 5760
   gcatcgccga cttcgaccgc ggcgtcttca agggcatcct ccagggcacc gacatcgccg 5820
   ggcctctcgt cgtctacccg ctcaacaaat ccaagtacgc actacgcgtc catcgtgcat 5880
   gcatgcatgc gatcctgcat ggtatgtatc acgcacttaa ctgacactgg ctcggtcgtg 5940
   gtttcaggtg ggacgacggc atgtcggcgg tgacgccagc ggaggaggtg ttctacgcgg 6000
   tgtcgctgcc cttctcgtcg gtggccaacg acctgaagcg gctggaggcg cagaaccaga 6060
   agatactgcg gttctgcgac ctcgccgggg tagggtacaa ggagtacctg gcgcactaca 6120
   cggcccacgg cgactgggtc cggcacttcg gcggcaagtg gcagcgcttc gtggagatga 6180
   aggacaagta cgaccccaag aggctgctct ccccaggcca ggacatccct aggaaggtat 6240
   acatatatgt ttataattct ttgtttcccc tcttattcag atcgatcaca tgcatctttc 6300
   attgctcgtt tttccttaca agtagtctca tacatgctaa tttctgtaag gtgttgggct 6360
   ggaaattaat taattaatta attgacttgc caagatccat atatatgtcc tgatattaaa 6420
   tcttcgttcg ttatgtttgg ttaggctgat caatgttatt ctagagtcta gagaaacaca 6480
   cccaggggtt ttccaactag ctccacaaga tggtgggcta gctgacctag atttgaagtc 6540
   tcactcctta taattatttt atattagatc attttctaat attcgtgtct ttttttattc 6600
   tagagtctag atcttgtgtt caactctcgt taaatcatgt ctctcgccac tggagaaaca 6660
   gatcaggagg gtttattttg ggtataggtc aaagctaaga ttgaaattca caaatagtaa 6720
   aatcagaatc caaccaattt tagtagccga gttggtcaaa ggaaaatgta tatagctaga 6780
   tttattgttt tggcaaaaaa aaatctgaat atgcaaaata cttgtatatc tttgtattaa 6840
   gaagatgaaa ataagtagca gaaaattaaa aaatggatta tatttcctgg gctaaaagaa 6900
   ttgttgattt ggcacaatta aattcagtgt caaggttttg tgcaagaatt cagtgtgaag 6960
   gaatagattc tcttcaaaac aatttaatca ttcatctgat ctgctcaaag ctctgtgcat 7020
   ctccgggtgc aacggccagg atatttattg tgcagtaaaa aaatgtcata tcccctagcc 7080
   acccaagaaa ctgctcctta agtccttata agcacatatg gcattgtaat atatatgttt 7140
   gagttttagc gacaattttt ttaaaaactt ttggtccttt ttatgaacgt tttaagtttc 7200
   actgtctttt tttttcgaat tttaaatgta gcttcaaatt ctaatcccca atccaaattg 7260
   taataaactt caattctcct aattaacatc ttaattcatt tatttgaaaa ccagttcaaa 7320
   ttcttttagg ctcaccaaac cttaaacaat tcaattcagt ggagctcaga gcgatcgcag     7380
   acccggggat gtcctggcct ggggagagca gcctcttggg gtcgtacttg tccttcatct     7440
   ccacgaagcg ctgccacttg ccgccgaagt gccggaccca gtcgccgtgg gccgtgtagt     7500
   gcgccaggta ctccttgtac cctaccccgg cgaggtcgca gaaccgcagt atcttctggt     7560
   tctgcgcctc cagccgcttc aggtcgttgg ccaccgacga gaagggcagc gacaccgcgt     7620
   agaacacctc ctccgctggc gtcaccgccg acatgccgtc gtcccacctg aaaccacgac     7680
   cgagccagtg tcagttaagt gcgtgataca taccatgcag gatcgcatgc atgcatgcac     7740
   gatggacgcg tagtgcgtac ttggatttgt tgagcgggta gacgacgaga ggcccggcga     7800
   tgtcggtgcc ctggaggatg cccttgaaga cgccgcggtc gaagtcggcg atgcgggagc     7860
   ggggcacgag cacgatgagc caagggtgcg ggacacgcca cagcccgatc ttgtccagcg     7920
   ccccctcctc gccgtgcacc cggtccagga actccaggta cgacgcgtcc cgcacgaacg     7980
   cgtgcccctc ctcgtgcctc agcgtcgcca gcaccgagct gagctcctgg tccacggacg     8040
   cggccgtggc gctgtcgtag ttgagcgtcg tctcgatgac gtacacggtg gtagcgttcc     8100
   tcgccgcggc gacggccacg atcctggcga cgtcggcgtc ggtgaagaac ccgcccgagt     8160
   tcctcacccg gcggccacta gtgggtctag agtcctgctt taatgagata tgcgagacgc     8220
   ctatgatcgc atgatatttg ctttcaattc tgttgtgcac gttgtaaaaa acctgagcat     8280
   gtgtagctca gatccttacc gccggtttcg gttcattcta atgaatatat cacccgttac     8340
   tatcgtattt ttatgaataa tattctccgt tcaatttact gattgtaccc tactacttat     8400
   atgtacaata ttaaaatgaa aacaatatat tgtgctgaat aggtttatag cgacatctat     8460
   gatagagcgc cacaataaca aacaattgcg ttttattatt acaaatccaa ttttaaaaaa     8520
   agcggcagaa ccggtcaaac ctaaaagact gattacataa atcttattca aatttcaaaa     8580
   gtgccccagg ggctagtatc tacgacacac cgagcggcga actaataacg ctcactgaag     8640
   ggaactccgg ttccccgccg gcgcgcatgg gtgagattcc ttgaagttga gtattggccg     8700
   tccgctctac cgaaagttac gggcaccatt caacccggtc cagcacggcg gccgggtaac     8760
   cgacttgctg ccccgagaat tatgcagcat ttttttggtg tatgtgggcc ccaaatgaag     8820
   tgcaggtcaa accttgacag tgacgacaaa tcgttgggcg ggtccagggc gaattttgcg     8880
   acaacatgtc gaggctcagc aggacc                                          8906
<210> 14
   <211> 741
   <212> DNA
   <213> artificial sequence
<220>
   <223> sequence of the inserted TaCKX1 gene fragment, sense orientation
<400> 14
   tgaggaactc gggcgggttc ttcaccgacg ccgacgtcgc caggatcgtg gccgtcgccg       60
   cggcgaggaa cgctaccacc gtgtacgtca tcgagacgac gctcaactac gacagcgcca      120
   cggccgcgtc cgtggaccag gagctcagct cggtgctggc gacgctgagg cacgaggagg      180
   ggcacgcgtt cgtgcgggac gcgtcgtacc tggagttcct ggaccgggtg cacggcgagg      240
   agggggcgct ggacaagatc gggctgtggc gtgtcccgca cccttggctc atcgtgctcg      300
   tgccccgctc ccgcatcgcc gacttcgacc gcggcgtctt caagggcatc ctccagggca      360
   ccgacatcgc cgggcctctc gtcgtctacc cgctcaacaa atccaagtac gcactacgcg      420
   tccatcgtgc atgcatgcat gcgatcctgc atggtatgta tcacgcactt aactgacact      480
   ggctcggtcg tggtttcagg tgggacgacg gcatgtcggc ggtgacgcca gcggaggagg      540
   tgttctacgc ggtgtcgctg cccttctcgt cggtggccaa cgacctgaag cggctggagg      600
   cgcagaacca gaagatactg cggttctgcg acctcgccgg ggtagggtac aaggagtacc      660
   tggcgcacta cacggcccac ggcgactggg tccggcactt cggcggcaag tggcagcgct      720
   tcgtggagat gaaggacaag t                                                741
<210> 15
   <211> 36
   <212> DNA
   <213> artificial sequence
<220>
   <223> primer CKX2s
<400> 15
   ttcggaccga ctagtgaggc gaactctgga taaatg 36
<210> 16
   <211> 36
   <212> DNA
   <213> artificial sequence
<220>
   <223> CKX2a
<400> 16
   ttcctaggga gctcaaactg acccagacca ccaaga 36
<210> 17
   <211> 37
   <212> DNA
   <213> artificial sequence
<220>
   <223> primer HCV-F
<400> 17
   ttcggaccga ctagtatccc tggctcaacg tgctcgt 37
<210> 18
   <211> 37
   <212> DNA
   <213> artificial sequence
<220>
   <223> primer HCV-R
<400> 18
   ttcctaggga gctcagttga agatgtcttg gcccggg 37
<210> 19
   <211> 37
   <212> DNA
   <213> artificial sequence
<220>
   <223> primer TAC-F
<400> 19
   ttcggaccga ctagttgagg aactcgggcg ggttctt 37
<210> 20
   <211> 37
   <212> DNA
   <213> artificial sequence
<220>
   <223> primer TAC-R
<400> 20
   ttcctaggcc cgggacttgt ccttcatctc cacgaag 37
<210> 21
   <211> 20
   <212> DNA
   <213> artificial sequence
<220>
   <223> primer qOCS1
<400> 21
   cgagcggcga actaataacg 20
<210> 22
   <211> 20
   <212> DNA
   <213> artificial sequence
<220>
   <223> primer qOCS2
<400> 22
   aattctcggg gcagcaagtc 20
<210> 23
   <211> 20
   <212> DNA
   <213> artificial sequence
<220>
   <223> qOCS3
<400> 23
   cgagcggcga actaataacg 20
<210> 24
   <211> 20
   <212> DNA
   <213> artificial sequence
<220>
   <223> primer qOCS4
<400> 24
   aattctcggg gcagcaagtc 20
<210> 25
   <211> 24
   <212> DNA
   <213> artificial sequence
<220>
   <223> qOCS5
<400> 25
   gccgtccgct ctaccgaaag ttac 24
<210> 26
   <211> 20
   <212> DNA
   <213> artificial sequence
<220>
   <223> primer qOCS6
<400> 26
   caaaattcgc cctggacccg 20
<210> 27
   <211> 25
   <212> DNA
   <213> artificial sequence
<220>
   <223> primer pM1
<400> 27
   tcattcatct gatctgctca aagct 25
<210> 28
   <211> 25
   <212> DNA
   <213> artificial sequence
<220>
   <223> primer pM2
<400> 28
   tctcgcatat ctcattaaag cagga 25
<210> 29
   <211> 24
   <212> DNA
   <213> artificial sequence
<220>
   <223> primer pM3
<400> 29
   atgtccattc gaattttacc gtgt 24
<210> 30
   <211> 25
   <212> DNA
   <213> artificial sequence
<220>
   <223> primer pM4
<400> 30
   gatcagccta accaaacata acgaa 25
<210> 31
   <211> 22
   <212> DNA
   <213> artificial sequence
<220>
   <223> primer pM5
<400> 31
   ctcaaagctc tgtgcatctc cg 22
<210> 32
   <211> 21
   <212> DNA
   <213> artificial sequence
<220>
   <223> primer pM6
<400> 32
   ttattagttc gccgctcggt g 21
<210> 33
   <211> 25
   <212> DNA
   <213> artificial sequence
<220>
   <223> primer qCKX11
<400> 33
   tcgtcgtcta cccactcaac aaatc 25
<210> 34
   <211> 24
   <212> DNA
   <213> artificial sequence
<220>
   <223> primer qCKX12
<400> 34
   ttggggtcgt acttgtcctt catc 24
<210> 35
   <211> 24
   <212> DNA
   <213> artificial sequence
<220>
   <223> primer qCKX21
<400> 35
   ggcgaactct ggataaatgt cttg 24
<210> 36
   <211> 25
   <212> DNA
   <213> artificial sequence
<220>
   <223> primer qCKX22
<400> 36
   agttctgttc tggtgagcaa gtgac 25

## Description

The subject of the present invention is a DNA cassette, a binary vector, a strain of *A*. *tumefaciens* and a method of obtaining a cereal plant with increased productivity.

In the solution disclosed in international patent application WO02005EP06620 (Schmulling T., Werner T.) the authors achieved an increase in the productivity of plant seeds through the expression of cytokine oxidase in the aleuron layer and/or in the seed embryo as well as demonstrating expression vectors containing nucleic acids encoding cytokinin oxidase of *Arabidopsis thaliana* under the control of a tissue specific promoter warranting expression in the aleuron and/or the seed embryo. In the solutions described in the published patent applications WO03/050287 and US2005/004-4594, the same authors show a method of stimulating the growth and/or enlargement of the formation of side roots or sucker shoots through the expression of cytokine oxidase or another proteins, reducing the level of active cytokine genes in plants or their parts. Furthermore, they deliver a method of increasing seed size and/or mass, embryo size and/or mass through the expression of cytokinin oxidase or another protein which reduces the levels of active cytokine genes in all plants or in their parts. The goal of the present invention is to deliver a method and tools for its embodiment which facilitate the increased productivity of cereal plants through increasing the number and mass of seeds and/or increasing root mass.

Unexpectedly, the above stated goal was attained using the solution according to the present invention.

The subject of the present invention is a DNA cassette for increasing the productivity of cereal plants and/or root mass comprising the following elements:
a) an expression promoter,
b) a DNA fragment from a coding or non-coding portion of the cereal CKX cytokinin oxidase gene in a sense orientation,
c) an intron,
d) a DNA fragment from a coding or non-coding portion of the cereal CKX cytokinin oxidase gene in an antisense orientation and
e) a 3' transcription terminator,
wherein the cereal cytokinin oxidase gene is selected from among genes expressed in the developing head and/or root from a group encompassing the genes *HvCKX, TaCKX, ZmCKX, ScCKX* and *AsCKX,*
wherein expression of said DNA cassette leads to the formation of hpRNA, and then siRNA for silencing the expression of at least one *CKX* gene, and in effect the expression of CKX is depressed.

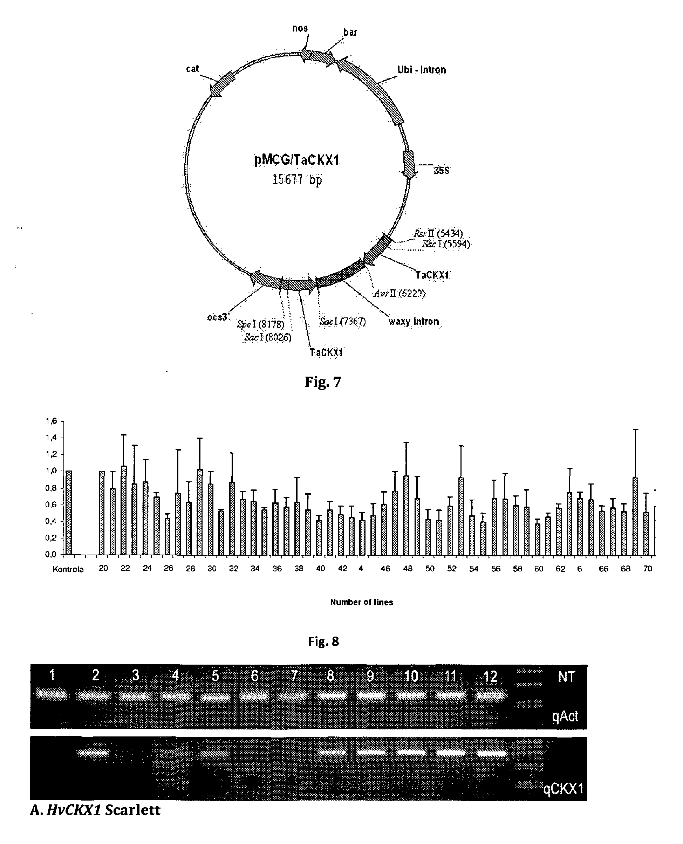

The term expression promoter according to the present invention can encompass any promoter active in cereal cells. In particular, this can be a constitutive or induced promoter, or a tissue- or development-specific promoter. According to the present invention, the "fragment of the cereal CKX cytokine oxidase gene" can comprise a sequence of at least 21 nucleotides being a fragment of a coding and/or non-coding sequence of the selected CKX gene whose expression occurs in developing heads and/or roots. In particular, this concerns the example gene *HvCKX1,* whose expression occurs in young roots, inflorescences and developing heads/grain (Figure 8) and *TaC KX1,* whose expression has been observed in grain and drought-stressed seedlings (Galuszka et al. 2004). This also relates to homologues and homelogues of genes of the CKX family in maize, *Zea mays* L., or *ZmCKX* text, rye, L., or *Secale cerea* L., *ScCKX* or oats, of *Avena sativa* L., or *AsCKX* which are expressed in developing heads and/or roots.

In particular, the 3'UTR of the above-mentioned CKX genes can also be the silencing signal. Preferentially, a cassette according to the present invention contains a fragment of the CKX cytokinin oxidase gene possessing a nucleotide sequence selected from among a group encompassing: Seq. Id. No. 1, Seq. Id. No. 3, Seq. Id. No. 4, Seq. Id. No. 5 or a fragment thereof. Furthermore, these may be coding sequences or fragments of cereal CKX genes available from the NCBI database under the numbers CA 031729, CA 705202, DQ903062, DQ 235927, DQ 238832, CA 603337, DJ 316444 and BJ 322935.

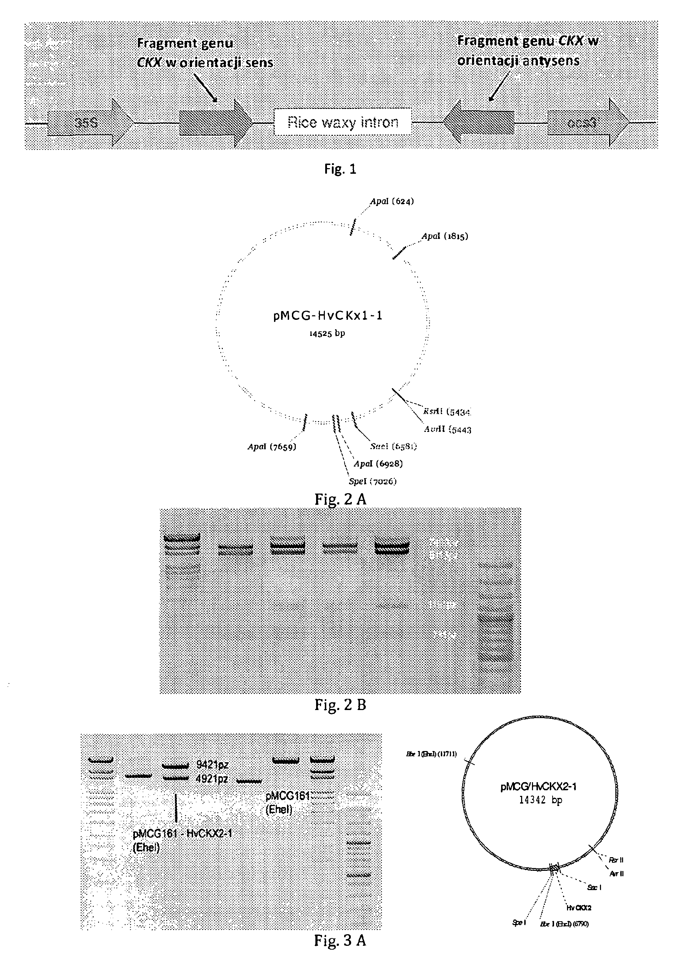

Preferentially, a silencing cassette according to the present invention contains a nucleotide sequence shown as Sequence 6 or Sequence 8. This can also be a silencing cassette containing a fragment of another cereal CKX gene which is expressed in developing heads and/or roots, in a sense and anti-sense orientation as shown in Figure 1.

The next subject of the present invention is a binary vector containing the DNA cassette defined above.

The next subject of the present invention is a strain of *A. tumefaciens* containing the binary vector defined above.

The next subject of the present invention is a method of obtaining a cereal plant with increased productivity characterized in that:
a) a DNA cassette according to the present invention, defined above, is produced, whereafter it is placed under the control of a promoter active in the modified cell, b) the resulting DNA cassette is introduced into the genome of a cereal plant, c) a cereal plant with increased productivity is regenerated from the resulting cell and/or d) a cereal plant with increased root mass is regenerated from the resulting cell.

Preferentially, during stage b) the transformation of the cereal plant cell is performed using a strain of *A. tumefaciens* containing a binary vector according to the present invention as defined above.

Unexpectedly, it turned out that a significant increase in productivity and root mass of cereal plants was caused by the depression of the expression of the *HvCKX1* gene and the inhibition of the activity of the CKX enzyme in these plants, which can be obtained via the introduction into their genome of an expression cassette encoding *hpRNAi* which is used to silence the gene encoding the enzyme CKX in cereal. Contrary to the suggestions stemming from the prior art cited at the beginning, and according to the present invention the increase of the productivity and root mass of cereal crops was unexpectedly achieved through a totally contrary procedure: the silencing of the expression of particular genes from the CKX family in barley and wheat. The increased productivity (seed mass and number) is positively correlated with root mass. This effect was achieved through the use of a constitutive promoter warranting the expression of the silencing cassette throughout the plant. The expression of the disclosed cassettes/cassette silenced the expression of particular cereal CKX gene/genes, and by the same token reduced the cytokine gene oxidase enzyme expression which led to the increase (and not the reduction) of the level of active cytokine genes in plants and/or their fragments.

### Figures

The present description has been supplemented with the attached figures.
Figure 1. Schematic representation of the silencing cassette containing fragments of the selected cereal CKX gene in a sense and anti-sense orientation.
Figure 2A. Cloning of the vector pMCG161-*HvCKX1.* A pal restriction sites in the cloned pMCG161 vector containing the first insert of a fragment of the *HvCKX1* gene.
Figure 2B. Cloning of the vector pMCG161-*HvCKX1*. Restriction analysis of the pMCG161 vector containing the second insert of the *HvCKX1* gene fragment using the enzyme ApaI.
Figure 3A. Cloning of the vector pMCG161-*HvCKX1.* Restriction analysis of the vector pMCG161 contain the first insert of the *HvCKX2* gene fragment using the enzyme DheI.
Figure 3B. Map of the vector pMCG161- *HvCKX2.* Restriction analysis of the vector pMCG161 containing both inserts of the *HvCKX2* gene fragment using the enzyme Ehe1.
Figure 4A. Cloning of the vector pMCG161-*TaCKX1*. Restriction analysis of the vector pMCG1 61 containing the first insert of the *TaCKX1* gene fragment using the enzyme SEC one.
Figure 4B. Cloning of the vector pMCG161-*TaCKX1*. Restriction analysis of the vector pMCG1 61 containing both inserts of the *TaCKX1* one gene fragment using the enzyme SEC I.
Figure 5. Structure of the vector pMCG/*HvCKX1*.
Figure 6. Structure of the vector pMCG/*HvCKX2*.
Figure 7. Structure of the vector pMCG/*TaCKX1*.
Figure 8. Relative activity of the enzyme cytokine oxidase/dehydrogenase (CKX) in the roots of T₁ saplings.

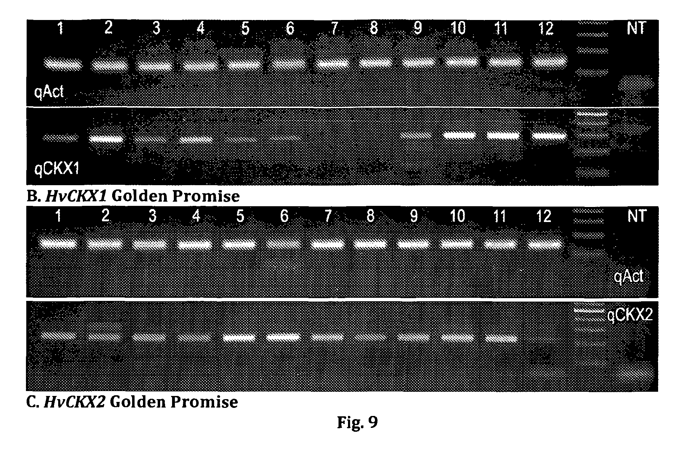

Figure 9A, B, and C. The results of a semi quantitative analysis of the expression of the gene *HvCKX1* in various tissues of the strain Scarlett (8) and golden promise (B) as well as *HVAC KX two* in Golden Promise (C). The upper portion of the gel represents the amplification of the cDNA of the reference gene, actin (qAct) and *HvCKX* is shown in the lower part (qCKX1, qCKX2). The consecutive lanes show the amplification of cDNA from: 1) 1-day seedlings about 1 cm long, 2) roots from 4 and 5 day seedlings, 3) meristem of 4 and 5 day seedlings, 4) the leaf of the 4 or 5 day seedling, 5) developing leaf of a 2-3 week plant, 6) a developed leaf of a 2-3 week plant, 7) a stem (along with a hypocotyl) from a 6 week plant, 8) early stage inflorescence (3-4 cm long), 9) older inflorescence (6-8 cm long), 10) head during pollination, 11) head a week following pollination, 12) head 2 weeks following pollination.

### Example 1. Production of hpRNAi vectors for silencing HvCKX1, HvCKX2 and ToCKX1 in cereal plants

To construct hpRNAi vectors we used the vector pMCG161 (http://www.chromdb.org/mcg161ohtml) containing a silencing cassette with cloning sites for the gene silencing fragment in a sense and anti-sense orientation. The cassettes were prepared based on the sequences of the genes *HvCKXl* (NCBI accession AF362472) and *HvCKX2* (NCBI accession AF540382) of barley as well as *ToCKXl* (NCBI accession AF362471) of wheat (Galuszka et al. , Eur. J. Biochem., 271: 3990-4002). These cassettes were composed of the following functional fragment: a CaMV 35S promoter (others may be used as well, particularly tissue-specific ones), fragment silencing gene in a sense orientation, intron Adhl, a fragment of the silencing gene in an antisense orientation as well as the OCS3' transcription terminator. The sequence of the silencing cassette containing the fragment of the *HvCKXl* gene is shown as Sequence 1. The sequence of the silencing cassette containing the fragment of the *HvCKX2* gene is shown as Sequence 2. The sequence of the silencing cassette containing the fragment of the *ToCKXl* gene is shown as Sequence 3. Following the transformation with a vector containing these cassettes they are integrated with the plant genome, and the siRNA they express regulates expression, by silencing the expression of the above genes.

Following cloning, the vectors were electroporated into *E. coli* (strain DH5a), isolated and analysed via restriction analysis using several enzymes. Vectors containing cloning constructs were then electroporated into *A*. *tumefaciens,* strain Agl1 and again tested using restriction analysis. A detailed description of the cloning to the vector and restriction analysis is shown below.

### Cloning

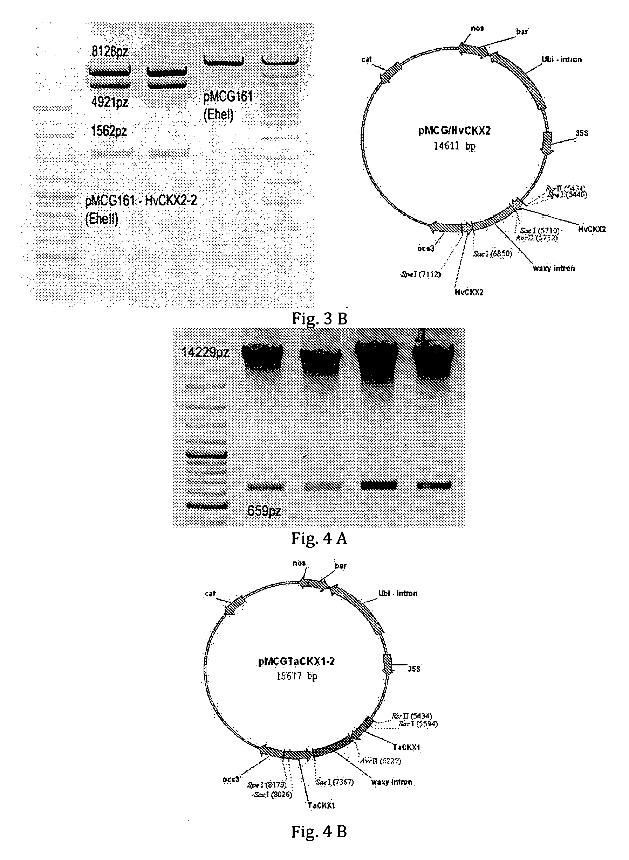

Preparation of pMCG161-HvCKX1. The stages of preparing the vector encompassed:
- Amplification of a fragment of HvCKXl (443 bp using primers with the sequences of restriction sites for *Sac*l, *Spe*l as well as *Rsrl*l and *Avrl*l. Amplification on vector pCRTl /NT-*TOPO-HvCKX1.*
- Restriction analysis with the restrictase *Apa*l.
- Purification of the amplified fragment using the Gene Clean KitII.
- Digestion of the vector pMCG161 and purified *HvCKX1* fragment the restrictase *Spe*l (*Bcu*l)*.*
- Purification of the vector and *HvCKXl* fragment with the Gene Clean Kit II.
- Digestion of the vector and fragment gene the restrictase *Sac*l.
- Purification of the digested vector and *HvCKXl* fragment with the GeneClean KitII
- Ligation of vector pMCG161 with the *HvCKXl* fragment (the vector:insert the molar ratio is 1:2).
- Purifcation of the ligation mixture with the GeneClean KitII
- Electroporation into E. coli (DH5a)
- Inoculation onto plates with an antibiotic (chloramphenicol - 35mg/ml).
- Reductive inoculation of selected colonies.
- Selected colonies were tested for the presence of the insert *HvCKX1,* using enzymatic digestion with *Apa*I. A scheme of the vector with the first cloned fragment of *HvCKX1* and ApaI restriction sites is shown in Fig. 2 A).
- After confirming the integration of the insert and vector pMCG161, we isolated the plasmid from a selected, positive colony.
- Digestion of the vector and insert as well as the *HvCKXl* fragment with the restrictases *Avr*II and *Rsr*ll
- Purification of the digested fragments.
- Ligation of the vector with a second *HvCKXl* fragment - vector: insert molar ratio is 1:2.
- Purification of the ligation mixture.
- Electroporation of the purified vector into E. coli (DH5a).
- Inoculation of bacteria onto selective medium (chloramphenicol 35mg/ml)
- Reductive inoculation of selected colonies.
- Confirmation of the presence of the integration of the second insert in the vector from selected colonies using restriction analysis with the restrictase *Apa*I. The restriction analysis is shown in Fig. 2B.
- Two-insert vectors were isolated from selected colonies.
- Electroporation of the vector pMCG161- HvCKXl into *Agrobacterium tumefaciens* (strain Agll)
- Inoculation of bacteria onto MG/L medium with rifampicine (50mg/l) and chloramphenicol (100mg/l); reductive inoculation of selected colonies as well as isolation of the plasmid from selected colonies.
- Restriction analysis of the vector isolated from *Agrobacterium* with the enzyme *Apa*I. Preparation of the vector pMCG161-*HvCKX2*
- Amplification of the fragment of *HvCKX2* (289 bp using primers with the sequences of restriction sites for *Sac*l, *Spe*l as well as *Rsr*ll and *Avrl*l. Amplification of the vector pDRIVE-*HvCKX2.*
- Confirmation of the amplified fragment with the restrictase *Ehe*l*.*
- Purification of the amplified fragment using the Gene Clean KitII.
- Digestion of the vector pMCG161 and purified *HvCKX2* fragment with the restrictases *Spe*l (*Bcu*l) and *Sac*l.
- Purification of the digested vector and *HvCKX2* fragment with the GeneClean KitII
- Ligation of the vector pMCG161 with the *HvCKX2* fragment (vector:insert molar ratio is 1:2)
- Purification of the ligation mixture with the GeneClean KitII
- Electroporation E. coli (DH5a)
- Inoculation of the bacteria onto antibiotic plates (chloramphenicol - 35mg/ml).
- Reductive inoculation of selected colonies.
- Selected colonies were checked for the presence of the *HvCKX2* insert using the restrictase *Ehe*l (Fig. 3 A).
- Following the confirmation of the integration of the insert with the vector pMCG161, we isolated the plasmid from a selected, positive colony.
- Digestion of the vector (and insert) as well as the *HvCKX2* fragment with the restrictases *Avr*II and *Rsr*II
- Purification of the digested fragments.
- Ligation of the vector with the second *HvCKX2* fragment - the vector:insert molar ratio is 1:2.
- Purification of the ligation mixture.
- Electroporation of the purified vector into E. coli (DH5a).
- Inoculation of bacteria onto selective medium (chloramphenicol 35mg/ml); reductive inoculation of selected colonies
- Confirmation of the integration of the second insert into the vector in selected colonies, using restriction analysis with the restrictase *Ehel* (Fig. 3 B).
- Two-insert vectors were isolated from selected positive colonies.
- Electroporation of the vector pMCG161-*HvCKX2* into *A*. *tumefaciens* (strain Agl1).
- Inoculation onto MG/L medium with rifampicine (50mg/l) and chloramphenicol (100mg/l).
- Reductive inoculation of selected colonies as well as isolation of the plasmid from selected colonies.
- Restriction analysis of the vector isolated from *Agrobacterium* with the restrictase *Ehe*l.
- Preparation of the vector pMCG161-TaCKX1
- Amplification of a fragment of *ToCKXl* (770 bp using primers containing the sequences of restriction sites for *Spel* and *Xma*l as well as *Rsrll* and *Avr*ll. Amplification onto the vector pDRIVE*-TaCKX1.*
- Confirmation of the amplified fragment with the restrictase *Sac*l.
- Purification of the amplified fragment using the Gene Clean KitII.
- Digestion of the vector pMCG161 and purified *ToCKXl* fragment with the restrictases *Spe*l (Bcul) and *Xma*l.
- Purification of the digested vector and *ToCKXl* fragment with the GeneClean KitII.
- Ligation of the vector pMCG161 and the *ToCKXl* fragment (vector:insert molar ratio is 1:2).
- Purification of the ligation mixture using GeneClean KitII.
- Electroporation into E. coli (DH5a)
- Inoculation onto antibiotic plates (chloramphenicol - 35mg/ml).
- Reductive inoculation of selected colonies.
- Selected colonies checked for the presence of the *TaCKX1* insert using digestion with the *Sac*I restrictase (Fig. 4 A).
- After checking the integration of the insert into the vector pMCG161, we isolated the plasmid with the insert from a selected, positive colony.
- Digestion vector (with insert) as well as ToCKXl fragment with restrictases Avrll and Rsrll.
- Purification of the digested fragment.
- Ligation of the vector z with the second *TaCKX1* fragment - vector:insert molar ratio is 1:2.
- Purification of the ligation mixture.
- Electroporation of the purified vector into E. coli (DH5a).
- Inoculation of the bacteria onto selection medium (chloramphenicol 35mg/ml); reductive inoculation of selected colonies.
- Checking the integration of the second insert into the vector in selected colonies with restriction analysis using the restrictase *Sac*I (Fig. 4 B).
- A two-insert vector was isolated from positive colonies.
- Electroporation vector pMCG161- ToCKXl to *A*. *tumefaciens* (strain Agl1)
- Inoculation of bacteria onto MG/L medium with rifampicine (50mg/l) and chloramphenicol (100mg/l); reductive inoculation of selected colonies and plasmid isolation from selected colonies.
- restriction analysis of the vector isolated from *Agrobacterium* using the restrictase *Sac*I.

### A. tumefaciens strains thus produced, containing appropriate binary vectors, were used to the transformation of cereal genes.

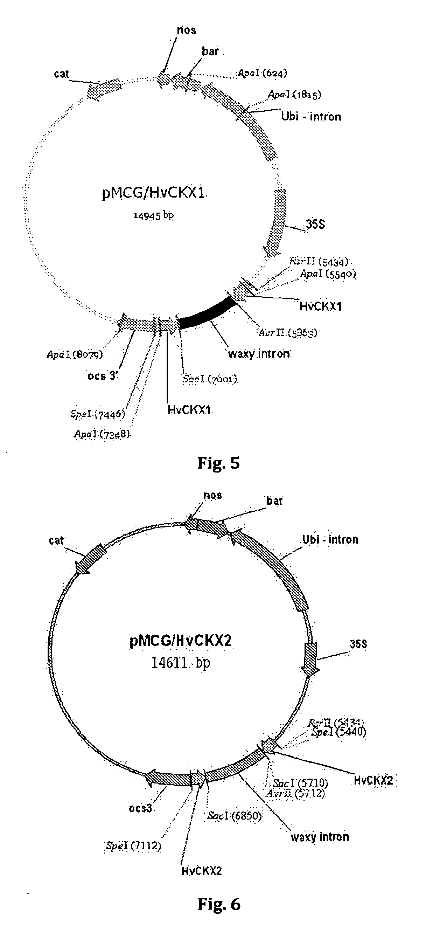

The binary vector pMCG/*HvCKX1* containing the following functional elements: T-DNA with a selection cassette as well as a silencing cassette is shown in Fig. 5, the nucleotide sequence of the T-DNA region of this vector is shown as Sequence 9, and the sequence of the inserted fragment of the gene *HvCKXl* in a sense orientation as Sequence 10. The binary vector pMCG/*HvCKX2* containing the following functional elements: T-DNA with a selection cassette as well as a silencing cassette is shown in Fig. 6, the nucleotide sequence of the T-DNA region of this vector is shown as Sequence 11, and the sequence of the inserted fragment of the gene *HvCKXl* in a sense orientation as Sequence 12. The binary vector pMCG/*TaCKX1* containing the following functional elements: T-DNA with a selection cassette as well as a silencing cassette is shown in Fig. 7, the nucleotide sequence of the T-DNA region of this vector is shown as Sequence 13, and the sequence of the inserted fragment of the gene *ToCKXl* in a sense orientation as Sequence 14.

To clone the gene fragments into the silencing cassettes, we used primers shown in Table 1.

**Table 1. Primer sequences designed and used to clone fragments for silencing genes in a sense and antisense orientation into a silencing cassette in the vector pMCG161.**

| Primer | Sequence | Use |
|---|---|---|
| CKX2s | | cloning cassette silencing gene *HvCKX2* |
| CKX2a | | |
| HCV-F | | cloning cassette silencing gene *HvCKXl* |
| HCV-R | | |
| TAC-F | | cloning cassette silencing gene *TaCKX1* |
| TAC-R | | |

### Example 2. Production of the transformed cereal plants

We transformed two strains of barley, Golden Promise and Scarlett, and wheat (Polish strains Torka and Kontesa) using a RNAi vector (via a gene modification method with the use of *Agrobacterium tumefaciens* as well as via a biolisitic method).

### In vitro culture method and transformation using A. tumefaciens

Extraction of barley and wheat embryos: heads 12-14 days following pollination (wheat) or 8-18 days post pollination (barley) are collected and then the grain is husked and sterilized. Seed sterilization: rinsing in 70% ethanol for a minute; decant alcohol, add 2-3 drops of Tween 20; immerse in 0.1% HgCl; rinse in sterilizing buffer for 3-4 minutes; rinse with sterile water 3 times, for 5, 10 and 15 minutes respectively; decant water; isolate and seed.

Seed 20 embryos on a plate with modified MSB3 medium for barley and MSB6 for wheat embryos.

MSB3 medium composition (modified acc. To Wan and Lemaux, 1994; Trifonova et al. 2001, Przetakiewicz et al. 2003): macro- and microelements acc. to Murashige and Skoog, (1962); 30 g/l maltose, 500 mg/l hydrolysed casein, 1.234 mg/l CuSO₄, 2.5 mg/l DICAMBA, 3.0 g/l GelRite, 0.02g/l thiamine, 5g/l myoinositol, 13.8g/l proline. pH 5.6 - 5.8

MSB6 medium composition (modified acc. to Przetakiewicz et al. 2003): the base MSB medium has macro and microelements according to Murashige and Skoog, (1962) and vitamins acc. To Gamborg et al. (1968). The medium MSB6 contains the components of MSB as well as: 30 g/l saccharose, 2 mg/l picrolam, 1 mg/l 2.4-3.0 g/l GelRite; pH 5.6 - 5.8.

The culture is maintained in a culture room at a temperature of 22-24°C, in a 16/8 photoperiod (day/night), 50 µEm-²s-¹ illumination under a filter tissue cover for 2-3 days. Preparation of the *A*. *tumefaciens* Agl1 strain for transformation - the bacterial culture is initiated sufficiently ahead of time (1 - 2 days). The culture is maintained in MG/L medium with the appropriate antibiotics (rifampicine 50mg/l, chloramphenicol - 70mg/l). When the culture reaches an appropriate stage (OD₆₀₀ = 0.6-1.2), the flask contents are transferred into centrifuge tubes, which are centrifuged for 10 minutes at 6000 rpm, 4°C. After centrifugation, the supernatant level is marked, and it is decanted. Fresh MSB3 medium is poured into the marked level. Acetosyringon is added. The tubes are placed on a shaker in order to dissolve the bacterial precipitate in the medium.

Transformation/inoculation of immature embryos with *A*. *tumefaciens -* The prepared bacterial suspension with acetosyringon is dropped onto each embryo. Two plates with untreated embryos are maintained (control). The culture is maintained in a culture room under standard conditions (as above), under lights and a cover of tissue.

Barley embryos were transformed with the *A*. *tumefaciens* Agl1 strain containing the vectors pMCG/*HvCKX1* and pMCG/*HvCKX2.* Wheat was transformed with *A*. *tumefaciens* Agl1 with the vectors pMCG161/*TaCKX1*, pMCG/*HvCKX1* and pMCG161/*HvCKX2.*

Passaging - After three days of post-inoculation with the bacteria, the embryos are transferred in groups of 6 onto a selection medium with the appropriate antibiotics (phosphinotricine-2mg/l, thimentin - 150 mg/l). At the same time, a positive control of the regeneration was maintained: untransformed embryos on non-antibiotic medium as well as a negative control: untransformed embryos on antibiotic medium. After four weeks we transferred embryos/callus lines in groups of 4 onto R2-MSB medium (Przetakiewicz et al. 2003) containing 1mg/l BA and 0.2 mg/l IAA with antibiotics as above. After another 2-4 weeks, the regenerating plants are transferred onto R2-MSB medium with antibiotics for further growth. The growing plants (over 1 cm) are transferred into 0.5 l jars and into 1/2 MS medium (half micro- and macroelement concentration acc. to Murashige and Skoog, 1962) with antibiotics. Successfully growing and rooting plants are planted into pots with fresh soil, and freshly planted plants are left for several days under cover to adapt them to the new conditions.

### Material for analysis can be collected from the growing plants.

We obtained 108 potentially transgenic plants from 75 callus lines. The results are shown in Table 2.

**Table 2. Numbers of transformation explants (immature embryos), selected plants as well as lines and transformation efficiency in the individual experiments using the silencing, control (pMCG161) and expression vector and via the Agrobacterium and biolistic methods.**

| Transformation using *A*. *Tumefaciens* | | | | | |
|---|---|---|---|---|---|
| strain | No. Exp./vector silencing and control | Number explants | Number of plants selected following the transformation | number of lines | Transformation efficiency (%) |
| Golden Promise | 1. pMCGICKX1 | 825 | 52 | 32 | 6.3 |
| | 4. pMCG/CKX2 | 421 | 36 | 28 | 8.6 |
| | 4.pMCG161 | 100 | 4 | 3 | 4.0 |
| | 5. pMCG/CKX2 | 75 | 5 | 4 | 6.7 |
| | 6. pMCG/CKX2 | 440 | 1 | 1 | 0.2 |
| | 6.pMCG161 | 75 | 0 | 0 | 0 |
| | 7. pMCG/CKX2 | 231 | 0 | 0 | 0 |
| | total | 2167 | 98 | 68 | 4.52 |
| Scarlett | 1. pMCG/CKX1 | 633 | 1 | 1 | 0.16 |
| | 4. pMCG/CKX2 | 507 | 1 | 1 | 0.20 |
| | 6. pMCG/CKX2 | 237 | 0 | 0 | 0 |
| | 6.pMCG161 | 125 | 0 | 0 | 0 |
| | 7. pMCG/CKX2 | 335 | 0 | 0 | 0 |
| | total | 1837 | 2 | 2 | 0.11 |
| Kontesa | 4. pMCG/CKX2 | 715 | 0 | 0 | 0 |
| | 4.pMCG161 | 100 | 0 | 0 | 0 |
| | 5. pMCG/*TaCKX1* | 76 | 0 | 0 | 0 |
| | 6. pMCG/*TaCKX1* | 322 | 0 | 0 | 0 |
| | 7. pMCG/*TaCKX1* | 71 | 0 | 0 | 0 |
| | total | 1284 | 0 | 0 | 0 |
| Torka | 4. pMCG/CKX2 | 550 | 0 | 0 | 0 |
| | 4.pMCG161 | 100 | 0 | 0 | 0 |
| | 5. pMCG/*TaCKX1* | 260 | 0 | 0 | 0 |
| | 6. pMCG/*TaCKX1* | 344 | 0 | 0 | 0 |
| | total | 1254 | 0 | 0 | 0 |
| Wanad | 4. pMCG/CKX2 | 1000 | 0 | 0 | 0 |
| | 4.pMCG161 | 125 | 0 | 0 | 0 |
| | 5. pMCG/*TaCKX1* | 350 | 0 | 0 | 0 |
| | total | 1475 | 0 | 0 | 0 |

| Biolistic transformation | | | | | |
|---|---|---|---|---|---|
| Golden Promise | 2. *HvCKX2*linear | 251 | 5 | 4 | 1.99 |
| Scarlett | 2. *HvCKX2*linear | 620 | 3 | 1 | 0.48 |

Phenotypic analysis of T₀ plants (genetically modified plants regenerated *in vitro*), production and analysis of T₁ progeny lines (from each T₀ one a line in which characteristics are inherited).

Seeds were obtained from all plants regenerated and selected on selection media. These were counted and weighed, and the mass per thousand seeds was calculated. The number of seeds ranged from 36 to 332 pieces, and the thousand seed mass (TSM) was from 12 to 41.28 g.

### Genetic analysis

Using genetic analyses (mainly PCR), we confirmed that the resulting T₀ are transgenic. For this purpose, we designed and used 7 pairs of specific primers, whose sequences are shown in Table 3.

**Table 3. Specific primers designed for the analysis of potentially transgenic plants selected after the following transformation.**

| Primer | Sequence | use |
|---|---|---|
| qOCS1 | CGAGCGGCGAACTAATAACG | qPCR (quantitative PCR) for the silencing cassette |
| qOCS2 | AATTCTCGGGGCAGCAAGTC | |
| qOCS3 | CGAGCGGCGAACTAATAACG | qPCR of the silencing cassette |
| qOCS4 | AATTCTCGGGGCAGCAAGTC | |
| qOCS5 | GCCGTCCGCTCTACCGAAAGTTAC | qPCR of the silencing cassette |
| qOCS6 | CAAAATTCGCCCTGGACCCG | |
| pM1 | TCATTCATCTGATCTGCTCAAAGCT | PCR of the silencing cassette |
| pM2 | TCTCGCATATCTCATTAAAGCAGGA | |
| pM3 | ATGTCCATTCGAATTTTACCGTGT | PCR of the silencing cassette |
| pM4 | GATCAGCCTAACCAAACATAACGAA | |
| pM5 | CTCAAAGCTCTGTGCATCTCCG | PCR of the silencing cassette |
| pM6 | TTATTAGTTCGCCGCTCGGTG | |

### Example 3. Silencing of the HvCKXl gene using hpRNA/siRNA leads to an increased productivity and root mass in cereal plants.

We analysed the gene silencing effect on *HvCKXl* as well as the phenotypic characteristics in 52 genetically modified lines of Golden Promise and 2 Scarlett lines.

The first stage of analyzing T₁ plants was to measure the activity level of the cytokinin oxidase/dehydrogenase enzyme (CKX) in the roots of plants resulting from transformation with a silencing vector for the gene *HvCKX1.* For this purpose, we sprouted groups of 5 of each T0 plant, cut off the root at the base, weighed them individually and pooled the roots from five plants for the measurements. The experiment was performed thrice (for 3 x 5 T₁ plants per line). The results of the relative activity of cytokinin oxidase/dehydrogenase (CKX) are shown with standard deviations for 52 analysed T₁ lines are shown in Fig. 8.

The relative values of these measurements, assuming the control measurement as 1.00 (line regenerated *in vitro*, not transformed) varied from 0.38 to 1.23. A significantly lower cytokinin oxidase/dehydrogenase activity level was noted in 40 lines. In order to compare the enzymatic activity with line productivity as well as root mass, they were divided into three groups: 1) with a relative CKX activity level below 0.59, 2) with a relative activity level from 0.6 to 0.79 and 3) above 0.8. The first two groups, encompassing 40 lines, exhibited a significantly lower enzymatic activity level in relation to the third group, which was similar to the control. A compilation of the results, encompassing seed number, thousand seed mass in T₀ plants as well as an average root mass (of the 15 progeny plants, T₁) and relative CKX activity in the roots is shown in Table 4.

**Table 4. Three groups of lines: with CKX enzymatic activity below 0.59; from 0.6 to 0.79 and above 0.8 and their corresponding productivity and root mass levels.**

| Enzymatic activity <0.59 | | | | | | |
|---|---|---|---|---|---|---|
| Line No. | T₀ plant | number of seeds | seed mass (mg) | thousand seed mass (g) | average root mass (mg) in T₁ | relative CKX activity |
| 25 | 2G/4 | 210 | 8522 | 40,58 | 40,21 | 0,44 ± 0,05 |
| 30 | 5G/2B | 63 | 2053 | 32,59 | 26,50 | 0,53 ± 0,02 |
| 34 | 5G/4 | 214 | 7567 | 35,36 | 27,95 | 0,54 ± 0,03 |
| 36 | 5G/5B | 185 | 6223 | 33,64 | 33,85 | 0,58 ± 0,11 |
| 38 | 5G17A | 203 | 6382 | 31,44 | 31,92 | 0,54 ± 0,19 |
| 39 | 5G/7B | 197 | 6467 | 32,83 | 30,92 | 0,41 ± 0,06 |
| 40 | 5G/8 | 217 | 6564 | 30,25 | 29,29 | 0,54 ± 0.1 |
| 41 | 5G/9 | 99 | 3221 | 32,54 | 31,33 | 0,49 ± 0.1 |
| 42 | 5G/10A | 184 | 4711 | 25,60 | 29,63 | 0,45±0,14 |
| 43 | 5G/10B | 239 | 7850 | 32,85 | 33,90 | 0,42 ± 0,08 |
| 44 | 5G/11 | 157 | 5031 | 32,04 | 37,80 | 0,48±0,15 |
| 49 | 5G/14A | 233 | 8470 | 36,35 | 33,00 | 0,43±0,12 |
| 50 | 5G/14B | 217 | 6725 | 30,99 | 32,92 | 0,43±0,12 |
| 51 | 5G/15A | 55 | 1381 | 25,11 | 34,13 | 0,59 ± 0,11 |
| 53 | 5G/16B | 120 | 4091 | 34,09 | 29,13 | 0,48±0,19 |
| 54 | 5G/17 | 142 | 3875 | 27,29 | 21,45 | 0,41 ±0,11 |
| 57 | 5G/19/C | 71 | 2305 | 32,46 | 31,88 | 0,59±0,12 |
| 58 | 5G/20A | 126 | 4061 | 32,23 | 37,40 | 0,58 ± 0,20 |
| 59 | 5G/20B | 217 | 6685 | 30,81 | 23,58 | 0,38 ± 0,06 |
| 60 | 5G/20C | 174 | 5743 | 33,01 | 27,79 | 0,46 ± 0,04 |
| 61 | 5G/20D | 177 | 6230 | 35,20 | 28,10 | 0,57 ± 0,05 |
| 65 | 5G/23B | 170 | 7018 | 41,28 | 27,83 | 0,53 ± 0,07 |
| 66 | 5G/24 | 142 | 4796 | 33,77 | 30,90 | 0,58 ± 0,11 |
| 67 | 5G/25 | 185 | 6080 | 32,86 | 30,25 | 0,53 ± 0,09 |
| 69 | 6G/1A | 143 | 3721 | 26,02 | 37,00 | 0,52 ± 0,23 |
| 70 | 6G11B | 235 | 7939 | 33,78 | 33,25 | 0,59 ± 0,20 |
| average | 26 | 168,27 | 5527,35 | 32,50 | 31,23 | 0,50 ± 0,11 |
| enzymatic activity 0.6 - 0.79 | | | | | | |
| 24 | 2G/3C | 90 | 2175 | 24,17 | 16,13 | 0,69 ± 0,06 |
| 27 | 5G/1B | 94 | 2542 | 27,04 | 18,50 | 0,63 ± 0,25 |
| 32 | 5G/3A | 86 | 3330 | 38,72 | 29,38 | 0,67 ± 0,09 |
| 33 | 5G/3B | 265 | 8678 | 32,75 | 30,13 | 0,65±0,14 |
| 35 | 5G/5A | 260 | 6993 | 26,90 | 25,33 | 0,63 ± 0,17 |
| 37 | 5G/6 | 195 | 7477 | 38,34 | 30,88 | 0,64 ± 0,29 |
| 45 | 5G/12A | 49 | 1476 | 30,12 | 32,20 | 0,62±0,15 |
| 46 | 5G/12A-1 | 166 | 5509 | 33,19 | 44,50 | 0,77 ± 0,23 |
| 48 | 5G/13 | 134 | 3847 | 28,71 | 30,25 | 0,68 ± 0,27 |
| 55 | 5G/18 | 202 | 7016 | 34,73 | 34,83 | 0,69±0,22 |
| 56 | 5G/19A | 177 | 6181 | 34,92 | 38,08 | 0,68±0,31 |
| 62 | 5G/21 | 206 | 7532 | 36,56 | 25,13 | 0,76 ± 0,28 |
| 63 | 5G/22 | 89 | 2608 | 29,30 | 24,05 | 0,68 ± 0,08 |
| 64 | 5G/23A | 148 | 5030 | 33,99 | 18,50 | 0,67 ± 0,19 |
| average | 14 | 154,36 | 5028,14 | 32,10 | 28,42 | 0,68 ±0,20 |

| enzymatic activity >0.8 | | | | | | |
|---|---|---|---|---|---|---|
| 19 | KOP 4G/1 | 50 | 489 | 9,78 | data n/a | 1,00±0,00 |
| 20 | 2G/1 | 36 | 432 | 12,00 | 9,83 | 0,80 ± 0,21 |
| 21 | 2G/2 | 66 | 1430 | 21,67 | 16,53 | 1,07 ± 0,37 |
| 22 | 2G/3A | 77 | 1786 | 23,19 | 27,00 | 0,85 ± 0,47 |
| 23 | 2G/3B | 80 | 2056 | 25,70 | 25,42 | 0,87 ± 0,27 |
| 28 | 5G11C | 145 | 3584 | 24,72 | 26,54 | 1,03 ± 0,37 |
| 29 | 5G/1D | 143 | 3116 | 21,79 | 18,00 | 0,85±0,15 |
| 31 | 5G/2B | 214 | 6530 | 30,51 | 24,04 | 0,88 ± 0,35 |
| 47 | 5G/12B | 100 | 3082 | 30,82 | 27,88 | 0,96 ± 0,40 |
| 52 | 5G116A | 177 | 5978 | 33,77 | 27,60 | 0,93 ± 0,38 |
| 68 | 5G/28 | 148 | 4850 | 32,77 | 19,45 | 0,93 ± 0,58 |
| 71 | 6G/2 | 110 | 3976 | 36,15 | 31,42 | 0,85 ± 0,40 |
| average | 12 | 117,82 | 3347,27 | 26,64 | 23,06 | 0,92 ± 0,33 |

A comparison of the averages of three groups shows a clear positive correlation between productivity and root mass in lines with depressed CKX activity. In the first group, encompassing plants with a relative enzymatic activity level below 0.59 (average = 0.50 ± 0.11). The average seed number in T₀ plants was 168.27; thousand seed mass was 32.5 g, and the average root mass of T₁ seedlings was 31.23 mg. In the second line, with the average relative activity level of 0.6 to 0.79 (average = 0.68 ±0.20) the values were, respectively: 154.36; 32.10g and 28.42 mg. In the third group, encompassing lines with activities approaching those of the control (average = 0.92 ± 0.33) we obtained on average 117.82 seeds, with a mass of 26.64 g and average root mass in T₁ seedlings of 23.06 mg. The results of decreased enzymatic activity levels in 40 transgenic lines of the Golden Promise strain of barley attest to, on average, a significantly decreased expression of the silenced gene *HvCKX1.* One cannot also discount the possibility of other genes from this family by the construct used, whose expression occurs in the root and which have sequences homologous to those used in the silencing construct. A consequence of the reduced activity of the cytokinin oxidase/dehydrogenase enzyme is an increase in root mass, and in a portion of the genes, an increase of the line's productivity (Table 4). The lower the CKX activity level, the higher the number of seeds obtained as well as higher thousand seed masses in T₀ plants as well as average root mass in T₁ plants.

The relative, quantitative measurement of the expression of the *HvCKXl* gene in transgenic T₁ plants of the Golden Promise strain, transformed with a silencing construct for this gene. During the second stage of analysis of T1 plants, we performed measurements of the expression of the *HvCKXl* gene in the roots of four-day seedlings. We sprouted 6 seeds from each T₀ line, individually cut off the root at the base and weighed it. RNA was isolated from a portion of the root and then transcribed into cDNA.

To perform the quantitative analysis of the expression of the *HvCKX2* gene, we designed and used the following primers:

| Primer | Sequence | use |
|---|---|---|
| qCKX11 | TCGTCGTCTACCCACTCAACAAATC | RT-PCR and qRT-PCR of the *HvCKXl* gene |
| qCKX12 | TTGGGGTCGTACTTGTCCTTCATC | |

the results of these measurements in selected T₁ plants are shown in Table 5.

**Table 5. relative quantitative measurement of the expression of the gene HvCKX1 in transgenic T₁ generation plants of the strain Golden Promise transformed with a construct for silencing this gene.**

| plant identifier | | plant | root mass (mg) | ground root material (mg) | RNA conc. (ng/ul) | efficiency of isolation from root (ng/mg) | rel. expression of CKX1 in the root MCt |
|---|---|---|---|---|---|---|---|
| 1 | GP/6 | Golden Prom. | 64 | 31 | 264,39 | 255,86 | 1.00^{a},d,e,f |
| 2 | GP/7 | Golden Prom. | 56 | 32 | 402,31 | 377,17 | 1.00^{b} |
| 4 | GP in vitro 1/6 | II FG KP/1A | 81 | 43 | 414,2 | 288,98 | 1.11^{a} |
| 5 | GP in vitro 1/7 | II FG KP/1A | 81 | 45 | 465,45 | 310,30 | 1.09^{b} |
| 6 | GP in vitro 1/8 | II FG KP/1A | 56 | 27 | 470,43 | 522,70 | 1.00^{c} |
| 7 | 28/6 | 5G/1C | 44 | 23 | 240,3 | 313,43 | 1.89^{a} |
| 8 | 28/7 | 5G/1C | 42 | 23 | 453,37 | 591,35 | 1.22^{b} |
| 10 | 28/9 | 5G/1C | 43 | 30 | 570,5 | 570,50 | 1.16^{d} |
| 12 | 28/11 | 5G/1C | 64 | 44 | 670,5 | 457,16 | 0.80^{f} |
| 14 | 30/7 | 5G/2B | 71 | 45 | 748,77 | 499,18 | 0.97^{b} |
| 16 | 30/9 | 5G/2B | 46 | 24 | 507,5 | 634,38 | 0.81^{d} |
| 19 | 38/6 | 5G/7A | 55 | 35 | 424,41 | 363,78 | 1.93^{a} |
| 20 | 38/7 | 5G/7A | 52 | 40 | 609,55 | 457,16 | 1.23^{b} |
| 25 | 43/6 | 5G/10B | 34 | 18 | 187,17 | 311,95 | 2.51^{a} |
| 26 | 43/7 | 5G/10B | 69 | 42 | 460,64 | 329,03 | 1.89^{b} |
| 27 | 43/8 | 5G/10B | 73 | 52 | 722,60 | 416,88 | 0.82^{c} |
| 31 | 52/6 | 5G/16A | 69 | 39 | 347,51 | 267,32 | 1.02^{a} |
| 32 | 52/7 | 5G/16A | 66 | 26 | 346,9 | 400,27 | 2.72^{b} |
| 35 | 52/10 | 5G/16A | 60 | 40 | 583,8 | 437,85 | 0.86^{e} |
| 36 | 52/11 | 5G/16A | 60 | 32 | 567,2 | 531,75 | 1.06^{f} |
| 37 | 59/6 | 5G/20B | 71 | 40 | 460,35 | 345,26 | 1.14^{a} |
| 38 | 59/7 | 5G/20B | 56 | 32 | 448,15 | 420,14 | 1.09^{b} |
| 42 | 59/11 | 5G/20B | 58 | 37 | 438,1 | 355,22 | 1.11^{f} |
| 43 | 70/6 | 6G/1B | 49 | 27 | 348,3 | 387,00 | 1.46^{a} |
| 44 | 70/7 | 6G/1B | 62 | 39 | 609,28 | 468,68 | 1.23^{b} |
| 45 | 70/8 | 6G/1B | 47 | 23 | 463,91 | 605,10 | 0.89^{c} |
| average mass per expression level > 0.80 | | | 58,81 | | | | |
| 9 | 28/8 | 5G/1C | 44 | 27 | 492,92 | 547,69 | 0.60^{c} |
| 18 | 30/11 | 5G/2B | 102 | 77 | 1009,9 | 393,47 | 0.61^{f} |
| 39 | 59/8 | 5G/20B | 45 | 21 | 638,86 | 912,66 | 0.64^{c} |
| 41 | 59/10 | 5G/20B | 37 | 17 | 525,5 | 927,35 | 0.6g^{e} |
| 28 | 43/9 | 5G/10B | 51 | 19 | 437,3 | 690,47 | 0.72^{d} |
| 30 | 43/11 | 5G/10B | 72 | 42 | 577,5 | 412,50 | 0.71^{f} |
| 34 | 52/9 | 5G/16A | 53 | 21 | 443 | 632,86 | 0.73^{d} |
| 48 | 70/11 | 6G/1B | 70 | 36 | 457,9 | 381,58 | 0.73^{f} |
| 17 | 30/10 | 5G/2B | 66 | 44 | 655,1 | 446,66 | 0.76^{e} |
| 24 | 38/11 | 5G/7A | 80 | 54 | 737,1 | 409,50 | 0.79^{f} |
| average mass per expression level 0.60 - 0,79 | | | 62,00 | | | | |
| 46 | 70/9 | 6G/1B | 44 | 15 | 312,7 | 625,40 | 0.10^{d} |
| 11 | 28/10 | 5G/1C | 82 | 58 | 714,8 | 369,72 | 0.31^{e} |
| 22 | 38/9 | 5G/7A | 85 | 59 | 786,2 | 399,76 | 0.41^{d} |
| 47 | 70/10 | 6G/1B | 71 | 52 | 737,6 | 425,54 | 0.45^{e} |
| 15 | 30/8 | 5G/2B | 57 | 34 | 699,95 | 617,60 | 0.51^{c} |
| 40 | 59/9 | 5G/20B | 42 | 19 | 420,6 | 664,11 | 0.54^{d} |
| 29 | 43/10 | 5G/10B | 80 | 52 | 604,2 | 348,58 | 0.54^{e} |
| 23 | 38/10 | 5G/7A | 68 | 41 | 783,3 | 573,15 | 0.57^{e} |
| 13 | 30/6 | 5G/2B | 99 | 57 | 517,77 | 272,51 | 0.58^{a} |
| 33 | 52/8 | 5G/16A | 42 | 18 | 390,08 | 650,13 | 0.59^{c} |
| 21 | 38/8 | 5G/7A | 58 | 40 | 719,82 | 539,87 | 0.59^{c} |
| average mass per expression level < 0,59 | | | 66,18 | | | | |

The T₁ plants tested in table 6 were segregated into three groups depending on the intensity of the silencing of the expression of the gene in question. Among T₁ plants exhibiting a relative expression level of *HvCKXl* in excess of 0.80, the average root mass in a 4-day seedling was 58.81 mg. Among plants with a relative expression from 0.60 to 0.79 the average root mass was 62.00 mg. In the group with the lowest relative expression of *HvCKXl* (below 0.59), the average root mass was 66.18 mg.

Conclusions: We demonstrated a positive correlation between productivity and root mass of the examined lines and the lowered expression of the *HvCKX1* gene as well as CKX enzymatic activity. In the first group, encompassing plants with a relative enzymatic activity below 0.59 (average = 0.50 ± 0.11), the average number of seeds in T₀ plants was 168.27; thousand seed mass was 32.5 g, and the average root mass in T₁ seedlings was 31.23 mg. In the second group, with a relative enzyme activity level from 0.6 to 0.79 (average = 0.68 ±0.20) the values were, respectively, 154.36, 32.10g and 28.42 mg. In the third group, encompassing lines with an activity level close to that of the control (average = 0.92 ± 0.33) we obtained on average 117.82 seeds with a mass of 26.64 g and an average root mass in T₁ seedlings of 23.06 mg. The results of the lowered enzymatic activity in 40 transgenic lines of Golden Promise barley match the results of the lowered expression level of the silenced gene *HvCKXl* in the examined plants. One cannot also discount the possibility of other genes from this family by the construct used, whose expression occurs in the root and which have sequences homologous to those used in the silencing construct. A consequence of the reduced expression of the *HVCKX1* gene is a reduction in the activity of cytokinin oxidase/dehydrogenase which leads to an increase in root mass, and in a portion of the genes, an increase of the line's productivity. The lower the CKX activity level, the higher the number of seeds obtained as well as higher thousand seed masses in T₀ plants as well as average root mass in T₁ plants.

### Productivity value

In plants exhibiting a relative CKX activity value, down to 0.5 (± 0.11), the number of seeds in T₀ plants grew to 142,8%; thousand seed mass to 122% and average root mass to 135.4%. In plants with a relative CKX activity level lowered to 0.69 (± 0.20) the number of seeds in T₀ plants grew to 131%; thousand seed mass to 120.5% an the average root mass to 123.2%. On the basis of this data, we can assume a productivity increase under field conditions of 106 - 120 % of the reference.

Example 4. Silencing of the *HvCKX2* gene using hpRNA/siRNA leads to decreased productivity and root mass in cereal plants.

This example shows that the silencing of cereal CKX genes expressed mainly in the somatic tissues (leaves) of cereal leads to an effect opposite of that claimed.

As a result of the biolistic transformation with a vector for silencing the gene *HvCKX2,* we obtained only 5 potentially transgenic Golden Promise plants and three Scarlett plants. The average relative CKX activity levels in the roots of T₁ seedlings of seven lines (triple analysis, 3 x 5 seedlings) ranged from 0.88 to 1.21 and were within the margin of error for the control plants. One of the Scarlett lines exhibited a significantly increased activity of 2.37 ± 0.02. The productivity data for these lines encompassing seed number, thousand seed mass, the relative root mass in T₁ seedlings as well as and relative CKX enzymatic activity are shown in Table 6. The productivity of control lines *in vitro* as well as root mass in both control strains, Scarlett and Golden Promise, was higher than in lines transformed with the construct for silencing the expression of *HvCKX2.*

**Table 6. Number of seeds and thousand seed mass (TSM) in T₀ plants as well as average root mass and average CKX activity in T₁ roots of a transformed line vector silencing gene HvCKX2.**

| seed number TSM (g) avg. root mass CKX activity std. deviation | | | | | | |
|---|---|---|---|---|---|---|
| | | 1953 | 39,06 | 36,38 | | |
| **Golden Promise** | II FG KP/1A | 275 | 32,86 | 41,60 | | |
| **1** | II FG KP/1B | 248 | 31,42 | 46,28 | | |
| **2** | II FG KP/1C | 198 | 32,16 | 46,00 | 1,00 | 0,00 |
| **3** | **average** | **240,33** | **32,15** | **44,63** | | |
| | II FG/1A | 292 | 32,22 | 30,92 | 0,93 | 0,29 |
| **4** | II FG/1B | 292 | 30,58 | 30,08 | 0,94 | 0,20 |
| **5** | II FG/2 | 190 | 29,02 | 54,88 | 0,88 | 0,12 |
| **6** | II FG/3 | 116 | 33,04 | 48,08 | 1,08 | 0,19 |
| **7** | II CG/1 | 55 | 9,68 | 22,00 | 1,21 | 0,00 |
| **8** | **average** | **189** | **26,91** | **37,19** | **1,01** | **0,16** |
| | | **2066** | **41,32** | **41,50** | | |
| **Scarlett** | BS KP/1 | 151 | 38,52 | 41,67 | | |
| **9** | BS KP/2A | 145 | 34,2 | 32,92 | | |
| **10** | BS KP/2B | 155 | 34,62 | 31,07 | 1,00 | 0,00 |
| **11** | BS KP/2C | 229 | 28,78 | 28,75 | | |
| **12** | FS KP/1 | 215 | 29,82 | 36,25 | | |
| **13** | FS KP/2 | 251 | 31,56 | 41,67 | | |
| **14** | **average** | **191** | **32,917** | **35,39** | | |
| | II FS 1A | 117 | 22,92 | 28,08 | 1,19 | 0,29 |
| **15** | II FS/1B | 307 | 26,2 | 25,00 | 1,02 | 0,34 |
| **16** | II FS/1C | 93 | 32,1 | 36,33 | 2,37 | 0,02 |
| | **average** | **172,33 27,07** | | **29,81** | **1,52** | **0,22** |

For the quantitative analysis of the expression of the *HvCKX2* gene we used the following primers:

| Primer | Sequence | use |
|---|---|---|
| qCKX21 | GGCGAACTCTGGATAAATGTCTTG | RT-PCR and qRT-PCR of the |
| qCKX22 | AGTTCTGTTCTGGTGAGCAAGTGAC | *HvCKX2* gene |

### Example 5. Analysis of the expression of native HvCKXl and HvCKX2 in various tissues of Golden Promise and Scarlett barley strains.

As an additional experiment we analysed the expression of the genes *HvCKXl* and *HvCKX2* in various tissues of control barley strains, Golden Promise and Scarlett (Fig. 9A, B, C). Literature data on this topic are very scant and insufficient for selecting appropriate tissues for analysis and interpretation of silencing results. As is evident from Fig. 9A and B, the high expression of *HvCKXl* in the tissues of control plants occurs in seedling roots and the inflorescences of the three studied stages, wherein it is highest in the head 7 days post pollination (7 DAP). The expression of the *HvCKX2* gene is evident in all 12 examined tissues (Fig. 9 C) wherein the highest amplification was noted in the developing and developed leaf of a 2-3 week old plant.

### Summary:

- We obtained 108 potentially transgenic lines of two strains of barley, Golden Promise and Scarlett, containing a silencing cassette against the genes *HvCKXl* and *HvCKX2* (as well as T-DNA without a silencing cassette as controls).
- We confirmed, using CKX enzymatic activity level measurements in the root, a very sharp decrease in enzymatic activity in almost 80 of the lines (40 from 52 tested lines) of the Golden Promise strain.
- We confirmed, using quantitative measurements of *HvCKXl* expression, the achieved effect of silencing the expression in transgenic lines; it was positively correlated with plant productivity and root mass.
- We showed that there is a strong correlation between a decreased CKX activity level and productivity (seed number and thousand seed mass) and root mass in lines with silencing.

We confirmed experimentally that the genetic modification method using a hpRNAi vector introduced via stable transformation into cereal facilitates:
- silencing of the activity of particular genes of the CKX family,
- function analysis,
- production of culture material with novel, preferential characteristics connected with the productivity of plants relating to seed mass and number, as well as the structure of the root system.

### Literature cited:

Galuszka P, Frebortova J, Werner T, Hamada M, Strnad M, Schmulling T, Frebort I. 2004. Cytokinin oxidase/dehydrogenase gene es in barley and wheat. Cloning and heterologous expression. Eur. J. Biochem. 271: 3990-4002.
Gamborg OL, Miller RA, Ojima K. 1968. Nutrient requirements of suspension cultures of soybean root cells. Exp. Celi Res. 50:.151-158.
Murashige T, Skoog F. 1962. A revised medium for rapid growth and bioassays with tobacco tissue cultures. Physiol. Plant. 15: 473-497.
Przetakiewicz A, Karas A, Orczyk W, Nadolska-Orczyk A. 2004. Agrobacterium-mediated transformation of polyploid cereal. The efficiency of selection and transgene expression in wheat. CelI. Mol. Biol. Lett. 9: 903-917.
Przetakiewicz A, Orczyk W, Nadolska-Orczyk A. 2003. The effect of auxin on plant regeneration of wheat, barley and triticale. Plant Celi, Tissue Org. Cult. 73: 245-256.
Wan Y, Lemaux PG. 1994. Generation of large num bers of independently transformation fertile barley plants. Plant Physiol. 104: 37-48.

## Claims

1. A DNA cassette for increasing the productivity of cereal plants and/or root mass comprising the following elements:
a) an expression promoter,
b) a DNA fragment from a coding or non-coding portion of the cereal CKX cytokinin oxidase gene in a sense orientation,
c) an intron,
d) a DNA fragment from a coding or non-coding portion of the cereal CKX cytokinin oxidase gene in an antisense orientation and
e) a 3' transcription terminator,
wherein the cereal cytokinin oxidase gene is selected from among genes expressed in the developing head and/or root from a group encompassing the genes *HvCKX, TaCKX, ZmCKX, ScCKX* and *AsCKX,*
wherein expression of said DNA cassette leads to the formation of hpRNA, and then siRNA for silencing the expression of at least one *CKX* gene, and in effect the expression of CKX is depressed.

2. A cassette according to Claim 1, **characterized in that** it contains a fragment of the CKX cytokinin oxidase gene possessing a nucleotide sequence selected from among a group encompassing: Seq. Id. No. 1, Seq. Id. No. 3, Seq. Id. No. 4, Seq. Id. No. 5 or a fragment thereof.

3. A cassette according to Claim 1, **characterized in that** it possesses a nucleotide sequence represented as Seq. Id. No. 6 or Seq. Id. No. 8.

4. A binary vector containing a DNA cassette defined in Claims 1 to 3.

5. A strain of *A. tumefaciens* containing a binary vector according to Claim 4.

6. A method of obtaining a cereal plant with increased productivity, **characterized in that**:
a) a DNA cassette defined by claims 1 to 3 is produced, whereafter it is placed under the control of a promoter active in the modified cell, b) the resulting DNA cassette is introduced into the genome of a cereal plant, c) a cereal plant with increased productivity is regenerated from the resulting cell and/or d) a cereal plant with increased root mass is regenerated from the resulting cell.

7. A method according to Claim 6, **characterized in that** during stage b) the transformation of the cereal plant cell is performed using a strain of *A. tumefaciens* containing a binary vector according to Claim 4.

## Patentansprüche

1. DNA-Kassette zur Erhöhung der Produktivität von Getreide-Pflanzen- und/oder Wurzelmasse, umfassend die folgenden Elemente:
a) einen Expressionspromotor,
b) ein DNA Fragment aus einem kodierenden oder nicht-kodierenden Teil des Getreide-CKX Cytokininoxidasegens in einer Sinn-Orientierung,
c) ein Intron,
d) ein DNA Fragment aus einem kodierenden oder nicht-kodierenden Teil des Getreide-CKX Cytokininoxidasegens in einer anti-Sinn-Orientierung, und
e) ein 3' Transkriptions-Terminator,
wobei das Getreide-Cytokininoxidasegen ausgewählt ist aus Genen, die in der sich entwickelnden Spitze und/oder der Wurzel exprimiert werden, aus einer Gruppe umfassend die Gene *HvCKX. TaCKX, ZmCKX,* ScCKXund *AsCKX,*
wobei die Expression der DNA-Kassette zur Bildung von hpRNA und dann siRNA zur Stummschaltung der Expression von mindestens einem CKX-Gen führt, und im Effekt die Expression von CKX unterdrückt wird.

2. Kassette nach Anspruch 1, **dadurch gekennzeichnet, dass** sie ein Fragment des CKX Cytokininoxidasegens enthält, das eine Nukleotidsequenz ausgewählt aus der Gruppe umfassend Seq. Id. No. 1, Seq. Id. No. 3, Seq. Id. No. 4, Seq. Id. No. 5 oder ein Fragment davon besitzt.

3. Kassette nach Anspruch 1, **dadurch gekennzeichnet, dass** sie eine als Seq. Id. No. 6 oder Seq. Id. No. 8 angegebene Nukleotidsequenz besitzt.

4. Binärer Vektor, enthaltend eine DNA-Kassette wie in den Ansprüchen 1 bis 3 definiert.

5. Stamm von *A. tumefaciens,* enthaltend einen binären Vektor nach Anspruch 4.

6. Verfahren zum Erhalt einer Getreide-Pflanze mit erhöhter Produktivität, **dadurch gekennzeichnet, dass**:
a) eine durch Ansprüche 1 bis 3 definierte DNA-Kassette wird hergestellt, wonach diese unter die Kontrolle eines in der modifizierten Zelle aktiven Promotors platziert wird, b) die sich ergebende DNA-Kassette wird in das Genom einer Getreide-Pflanze eingeführt, c) eine Getreide-Pflanze mit erhöhter Produktivität wird aus der sich ergebenden Zelle regeneriert, und/oder d) eine Getreide-Pflanze mit erhöhter Wurzelmasse wird aus der sich ergebenden Zelle regeneriert.

7. Verfahren nach Anspruch 6, **dadurch gekennzeichnet, dass** während der Phase b) die Transformation der Getreide-Pflanze unter der Verwendung eines Stammes von *A. tumefaciens,* enthaltend einen binären Vektor nach Anspruch 4 durchgeführt wird.

## Revendications

1. Cassette d'ADN pour augmenter la productivité de plantes céréalières et/ou masse racinaire comprenant les éléments suivants :
a) un promoteur d'expression,
b) un fragment d'ADN d'une partie codante ou non codante du gène de cytokinine oxydase CKX de céréale dans une orientation sens,
c) un intron,
d) un fragment d'ADN d'une partie codante ou non codante du gène de cytokinine oxydase CKX de céréale dans une orientation antisens et
e) un terminateurde transcription 3',
le gène de cytokinine oxydase étant choisi parmi des gènes exprimés dans la tête et/ou la racine en développement d'un groupe comprenant les gènes *HvCKX*, *TuCKX*, *ZmCKX*, *ScCKX* et *AsCKX*, l'expression de ladite cassette d'ADN conduisant à la formation d'ARNhp, et ensuite d'ARNsi pour le silençage de l'expression d'au moins un gène *CKX*, et effectivement à une réduction de l'expression de CKX.

2. Cassette selon la revendication 1, **caractérisée en ce qu'**elle contient un fragment du gène de cytokinine oxydase CKX possédant une séquence nucléotidique choisie dans un groupe comprenant : Seq Id No. 1, Seq Id No. 3, Seq Id No. 4, Seq Id No. 5 ou un fragment de ceux-ci.

3. Cassette selon la revendication 1, **caractérisée en ce qu'**elle possède une séquence nucléotidique représentée par Seq Id No. 6 ou Seq Id No. 8.

4. Vecteur binaire contenant une cassette d'ADN définie dans les revendications 1 à 3.

5. Souche de *A. tumefasciens* contenant un vecteur binaire selon la revendication 4.

6. Procédé d'obtention d'une plante céréalière ayant une productivité augmentée, **caractérisé en ce que** :
a) une cassette d'ADN définie par les revendications 1 à 3 est produite, après quoi elle est placée sous le contrôle d'un promoteur actif dans la cellule modifiée, b) la cassette d'ADN résultante est introduite dans le génome d'une plante céréalière, c) une plante céréalière ayant une productivité augmentée est régénérée à partir de la cellule résultante et/ou d) une plante céréalière ayant une masse racinaire augmentée est régénérée à partir de la cellule résultante.

7. Procédé selon la revendication 6, **caractérisé en ce que** pendant l'étape b), la transformation de la plante céréalière est effectuée en utilisant une souche de *A. tumefasciens* contenant un vecteur binaire selon la revendication 4.
